(19) Europäisches Patentamt
European Patent Office
Office européen des brevets

(11) Numéro de publication: **0 124 384**
**B1**

(12) # FASCICULE DE BREVET EUROPEEN

(45) Date de publication du fascicule du brevet: **05.08.87**

(21) Numéro de dépôt: **84400059.6**

(22) Date de dépôt: **12.01.84**

(51) Int. Cl.⁴: **C 07 D 513/04,** C 07 D 498/04, C 07 D 487/04, C 07 D 471/04, A 61 K 31/33

(54) **Nouveaux dérivés du pyrrole orthocondensé utiles pour la préparation de nouveaux médicaments et leur préparation.**

(30) Priorité: **13.01.83 FR 8300453**
**13.01.83 FR 8300454**

(43) Date de publication de la demande:
**07.11.84 Bulletin 84/45**

(45) Mention de la délivrance du brevet:
**05.08.87 Bulletin 87/32**

(84) Etats contractants désignés:
**AT BE CH DE FR GB IT LI LU NL SE**

(56) Documents cités:
**FR - A - 1 401 707**
**US - A - 3 642 807**

(73) Titulaire: **RHONE-POULENC SANTE, Les Miroirs 18 Avenue d'Alsace, F-92400 Courbevoie Cedex (FR)**

(72) Inventeur: **Fabre, Jean-Louis, 9, rue Fagon, F-75013 Paris (FR)**
Inventeur: **Farge, Daniel, 30, rue des Pins Sylvestres, F-94320 Thiais (FR)**
Inventeur: **James, Claude, 31 bis, rue Gambetta, F-75020 Paris (FR)**
Inventeur: **Lavé, Daniel, 72 Bld de la Villette, F-75019 Paris (FR)**

(74) Mandataire: **Le Goff, Yves et al, RHONE-POULENC RECHERCHES Brevets Pharma 25, Quai Paul Doumer, F-92408 Courbevoie (FR)**

**EP 0 124 384 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne de nouveaux dérivés du pyrrole orthocondensé de formule générale:

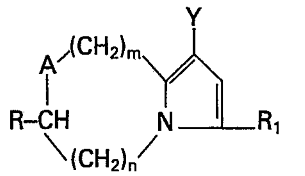

(I)

utiles pour la préparation de nouveaux médicaments ainsi que leurs sels.

Dans la formule générale (I),

a) soit Y représente un radical carboxy ou un radical de formule générale:

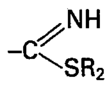

(II)

dans laquelle $R_2$ représente un radical alcoyle ou benzyle, A représente un atome de soufre, m représente le nombre 1, n représente le nombre zéro, R représente un radical pyridyle-3 et $R_1$ représente un atome d'hydrogène,

b) soit Y représente un radical cyano, carboxy ou un radical de formule générale (II) défini comme précédemment, A représente un atome de soufre ou d'oxygène ou un radical méthylène, m représente le nombre 1 ou 2 et n représente le nombre 0, 1 ou 2, étant entendu que la somme m + n est égale 1, 2 ou 3, R représente un atome d'hydrogène ou un radical alcoyle ou phényle (éventuellement substitué par un atome d'halogène ou un radical alcoyle, alcoyloxy ou trifluorométhyle) et $R_1$ représente un radical de formule générale:

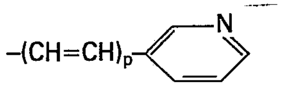

(III)

dans laquelle p représente le nombre zéro ou 1, étant entendu que, dans les définitions qui précèdent et celles qui suivront, les radicaux alcoyle ou portions alcoyle sont en chaîne droite ou ramifiée et contiennent, sauf mention spéciale, 1 à 4 atomes de carbone.

Selon l'invention, les produits de formule générale (I) dans laquelle Y représente un radical cyano et les autres symboles sont définis comme précédemment en b) c'est-à-dire les produits de formule générale:

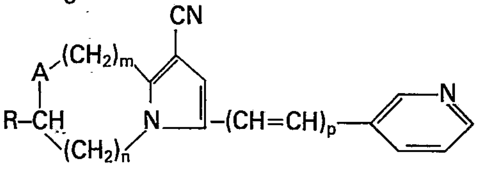

($I_A$)

peuvent être obtenus par action du chloro-2 acrylonitrile de formule:

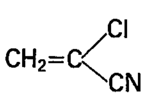

(IV)

sur un produit de formule générale:

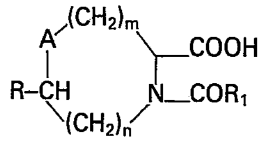

(V)

dans laquelle les différents symboles sont définis comme précédemment en b).

La réaction s'effectue généralement dans l'anhydride acétique par chauffage à une température comprise entre 80 et 130 °C.

Les produits de formule générale (V) dans laquelle A, R, $R_1$, m et n sont définis comme précédemment en b) peuvent être obtenus par condensation d'un produit de formule générale:

$R_1COZ_o$ (VI)

dans laquelle $R_1$ est défini comme précédemment en b) et $Z_0$ représente un atome d'halogène ou bien forme avec le radical ($R_1$CO– un anhydride mixte, sur un produit de formule générale:

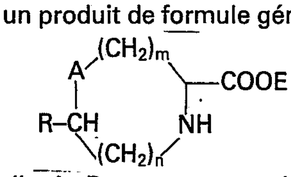

(VII)

dans laquelle A, R, m et n sont définis comme précédemment en b) et E représente un atome d'hydrogène ou un radical alcoyle, suivie, lorsque E représente un radical alcoyle, d'une hydrolyse.

La condensation du produit de formule générale (VI) sur le produit de formule générale (VII) s'effectue généralement dans un solvent organique inerte tel que le chloroforme en présence d'un accepteur d'acide tel que la triéthylamine à une température comprise entre 0 et 65 °C.

Lorsque E représente un radical alcoyle, l'hydrolyse s'effectue par toute méthode connue de l'homme du métier pour transformer un ester en acide sans toucher au reste de la molécule, notamment par chauffage en milieu alcalin dans l'eau ou un solvant hydro-alcoolique tel que le mélange eau-éthanol à une température comprise entre 20° et 80 °C.

Les produits de formule générale (VII) peuvent être obtenus par application ou adaptation des méthodes décrites par H.T. Nagasawa, J.A. Elberling, P.S. Fraser et N.S. Nizuno, J. Med. Chem. 14, 501 (1971) ou B. Belleau, J. Med. Chem. 2, 553 (1960) ou J.C. Wriston et C.G. McKenzie, J. Biol. Chem., 225, 607 (1957) ou S. Wolff, G. Militello et coll., Tet. Letters, 3913 (1979) ou H. Gershon et A. Scala, J. Org. Chem. 26, 2347 (1961) ou R. Riemschneider et G.A. Hoyer, Z. Naturforsch. 17B, 765 (1962) ou H. Möhrle et C. Karl, Arch. Pharm. 301, 728 (1968) ou R.K. Hill, T.H. Chan et J.A. Joule, Tetrahedron 21, 147 (1965).

Lorsque A représente un atome d'oxygène, n est égal à O, R et m sont définis comme précédemment en b), on n'isole pas le produit de formule générale (VII) mais on obtient directement le produit de formule générale (V), la condensation du produit de formule générale (VI) s'effectuant in situ dans le mélange réactionnel.

Selon l'invention, les produits de formule générale (I) dans laquelle Y représente un radical carboxy et les autres symboles sont définis comme précédemment peuvent être obtenus par hydrolyse d'un nitrile de formule générale:

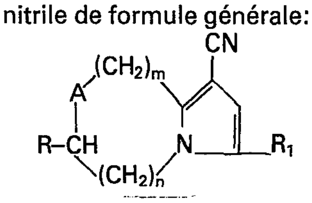

(VIII)

dans laquelle les symboles sont définis comme précédemment, par toute méthode connue de l'homme du métier pour transformer un nitrile en acide sans toucher au reste de la molécule, notamment par chauffage en milieu alcalin dans un alcool à haut point d'ébullition tel que l'éthylèneglycol, à une température comprise entre 100 °C et la température de reflux du mélange réactionnel.

Les produits de formule générale (VIII) dans laquelle les symboles sont définis comme précédemment en b), c'est-à-dire les produits de formule générale ($I_A$), peuvent être préparés comme il a été dit précédemment, c'est-à-dire par action d'un produit de formule (IV) sur un produit de formule générale (V).

Les produits de formule générale (VIII) dans laquelle les symboles sont définis comme précédemment en a) peuvent être préparés par condensation d'un produit de formule (IV) sur un produit de formule:

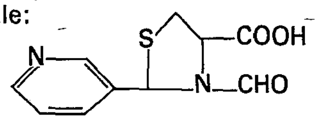

(IX)

La réaction s'effectue généralement dans l'anhydride acétique par chauffage à une température comprise entre 80 et 130 °C.

Le produit de formule générale (IX) peut être préparé par formylation du produit de formule:

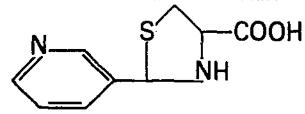

(X)

La formylation peut être avantageusement effectuée par action de l'acide formique dans l'anhydride acétique à une température comprise entre 10 et 25 °C.

Le produit de formule (X) peut être préparé selon la méthode de A. Banashek et M. I. Shchukina, J. Gen. Chem. U.S.S. R. 31, 1374 (1961); Chem. Abstr. 55, 24739 h (1961).

Selon l'invention, les produits de formule générale (I) dans laquelle Y représente un radical de formule générale (II) dans laquelle $R_2$ est défini comme précédemment et les autres symboles sont définis comme précédemment peuvent être préparés par action d'un halogénure de formule générale:

$R_2Z$ (XI)

dans laquelle $R_2$ est défini comme précédemment et Z représente un atome d'halogène, de préférence d'iode, sur un produit de formule générale:

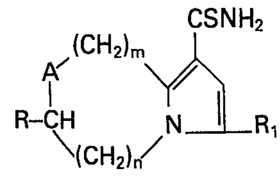

(XII)

dans laquelle les symboles sont définis comme précédemment.

On opère généralement dans un solvant organique tel que l'acétone ou un mélange d'acétone et de diméthylformamide à une température comprise entre 0 et 50 °C.

Les produits de formule générale (XII) peuvent être obtenus à partir d'un produit de formule générale (VIII) par toute méthode connue de l'homme du métier pour passer d'un nitrile à un thioamide sans toucher au reste de la molécule. Il est particulièrement avantageux de faire agir sur le nitrile de formule générale (VIII) le sulfure d'hydrogène dans un solvant tel que la pyridine, en présence de triéthylamine, en opérant à une température comprise entre 0 et 50 °C.

Les nouveaux produits de formule générale (I) sont utiles notamment comme produits intermédiaires pour la préparation de produits thérapeutiquement actifs de formule générale:

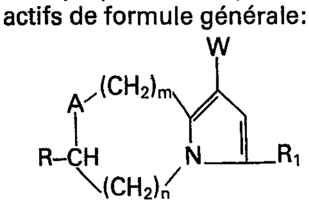

(XIII)

dans laquelle

A – ou bien m est égal à 1, n est égal à 0, R représente un radical pyridyle-3, $R_1$ représente un atome d'hydrogène et

$a_1$) soit W représente un radical acétyle,

$b_1$) soit W représente un radical de formule générale:

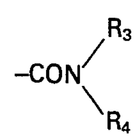

(XIV)

dans laquelle

– ou bien $R_3$ représente un atome d'hydrogène et $R_4$ représente un radical amino, alcoylamino, dialcoylamino, phénylamino ou diphénylamino,

– ou bien $R_3$ et $R_4$, identiques ou différents, représentent un atome d'hydrogène ou un radical alcoyle contenant 1 à 5 atomes de carbone ou phényle substitué,

– ou bien $R_3$ représente un atome d'hydrogène et $R_4$ représente un radical pyridyle ou alcoyle contenant 1 à 5 atomes de carbone substitué par un radical carboxy, amino, alcoylamino, dialcoylamino, morpholino, pipéridino, pyrrolidinyle-1, pipérazinyle-1 (éventuellement substitué en position 4 par un radical alcoyle, pyridyle, phényle éventuellement substitué ou benzyle éventuellement substitué), phényle éventuellement substitué, pyridyle ou imidazole

– ou bien $R_3$ et $R_4$ forment ensemble un radical

imidazolyle ou un hétérocycle à 5 ou 6 chaînons pouvant contenir en outre un autre hétéroatome tel que l'oxygène, le soufre ou l'azote éventuellement substitué par un radical alcoyle, alcoyloxycarbonyle, hydroxyalcoyle, aminoalcoyle, alcoylaminoalcoyle, dialcoylaminoalcoyle, pyridyle, pyrimidinyle, pyrazinyle, phényle éventuellement substitué ou benzyle éventuellement substitué,

$c_1$) soit W représente un radical de formule générale:

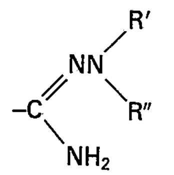

(XV)

dans laquelle R' et R'', identiques ou différents, représentent un radical alcoyle, étant entendu que dans les définitions précédentes les radicaux phényle et benzyle substitués sont les radicaux phényle et benzyle portant un atome d'halogène ou un radical alcoyle, alcoyloxy, alcoylthio, trifluorométhyle ou dialcoylamino.

B – ou bien m représente le nombre 1 ou 2 et n représente le nombre 0,1 ou 2, étant entendu que la somme m + n est égale à 1, 2 ou 3, A représente un atome de soufre ou d'oxygène ou un radical méthylène, R représente un atome d'hydrogène ou un radical alcoyle ou phényle (éventuellement substitué par un atome d'halogène ou un radical alcoyle, alcoyloxy ou trifluorométhyle), $R_1$ représente un radical de formule générale (III) défini comme précédemment et W représente un radical de formule générale:

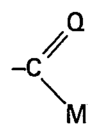

(XVI)

dans laquelle

$a_2$) soit Q représente un atome d'oxygène ou de soufre ou un radical imino et M représente un radical de formule générale:

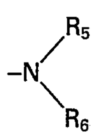

(XVII)

dans laquelle $R_5$ et $R_6$ représentent tous les deux un atome d'hydrogène ou bien $R_5$ représente un atome d'hydrogène et $R_6$ représente un radical hydroxy ou alcoyle contenant 1 à 5 atomes de carbone substitué par un radical carboxy, amino, alcoylamino, dialcoylamino, hydroxyalcoylamino, morpholino, imidazolyle, pipérazinyle-1 (éventuellement substitué en position 4 par un radical alcoyle, benzyle [éventuellement substitué par un atome d'halogène ou un radical alcoyle, alcoyloxy ou trifluorométhyle] ou phényle (éventuellement substitué par un atome d'halogène ou un radical alcoyle, alcoyloxy ou trifluorométhyle)], pipéridino, pyrrolidinyle-1 ou bien $R_6$ représente un radical phényle substitué par un ou plusieurs radicaux hydroxy, carboxy, amino, alcoylamino ou dialcoylamino, ou bien encore $R_5$ et $R_6$ forment avec l'atome d'azote auquel ils sont liés un cycle à 5 ou 6 chaînons pouvant en outre contenir un autre hétéroatome tel que l'oxygène, le soufre ou l'azote éventuellement substitué par un radical alcoyle, alcoyloxycarbonyle, hydroxyalcoyle, aminoalcoyle, alcoylaminoalcoyle, dialcoylaminoalcoyle, benzyle (éventuellement substitué par un atome d'halogène ou un radial alcoyle, alcoyloxy ou trifluorométhyle), pyrrolidinyl-1 carbonylalcoyle,

$b_2$) soit Q représente un radical dialcoylhydrazono, et M représente un radical amino,

$c_2$) soit Q et M forment avec l'atome de carbone auquel ils sont liés un radical △2-thiazolinyle-2 ou △2-imidazolinyle-2.

Pour obtenir les produits de formule générale (XIII) définis comme précédemment en A et dans laquelle W représente un radical de formule générale (XIV) dans laquelle $R_3$ et $R_4$ forment ensemble en cycle imidazolyle, on fait agir le N,N'-carbonyldiimidazole sur un produit de formule générale (I) dans laquelle Y représente un radical carboxy, $R_1$ représente un atome d'hydrogène, R représente un radical pyridyle-3, m est égal à 1 et n est égale à 0, c'est-à-dire un produit de formule:

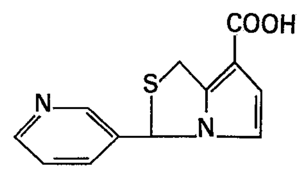

($I_B$)

On opère généralement dans un solvant organique inerte tel que le tétrahydrofuranne ou le diméthylformamide à une température voisine de 20 °C.

Pour obtenir les produits de formule générale (XIII) définis comme précédemment en A et dans laquelle W représente un radical de formule générale (XIV) dans laquelle $R_3$ et $R_4$ sont définis comme en $b_1$) à l'exception pour $R_3$ et $R_4$ de former ensemble un radical imidazolyle, on fait réagir l'ammoniac ou une amine de formule générale:

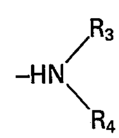

(XVIII)

dans laquelle $R_3$ et $R_4$ ont les définitions correspondantes sur un produit de formule générale (I) dans laquelle Y représente un radical carboxy, $R_1$ représente un atome d'hydrogène, R représente un radical pyridyle-3, m est égal à 1 et n est égal à 0, c-est-à-dire un produit de formule générale ($I_B$).

Lorsque $R_3$ ou $R_4$ représente un radical alcoyle contenant 1 à 5 atomes de carbone substitué par un radical amino, alcoylamino ou pipérazinyle ou bien que $R_3$ et $R_4$ forment ensemble avec l'atome

d'azote auquel ils sont liés un hétérocycle à 5 ou 6 chaînons contenant un autre atome d'azote éventuellement substitué par un radical aminoalcoyle, les fonctions amine correspondantes doivent être préalablement protégées avant condensation sur l'acide de formule générale ($I_B$).

Le blocage et le déblocage subséquent peuvent s'effectuer par toute méthode connue de l'homme du métier pour protéger une fonction amine primaire ou secondaire, par exemple sous forme de trifluoroacétamide, le déblocage étant réalisé à l'aide de méthanol ammoniacal.

Il est particulièrement avantageux d'utiliser l'acide de formule générale ($I_B$) sous une forme activée par exemple:

α) sous forme de chlorure d'acide; dans ce cas, on opère dans un solvant halogéné tel que le chloroforme, le chlorure de méthylène ou le dichloro-1,2 éthane ou un éther tel que le dioxanne, à une température comprise entre 20 °C et la température de reflux du mélange réactionnel, ou

β) sous forme d'anhydride mixte, obtenu par action d'un chloroformiate d'alcoyle sur l'acide de formule générale ($I_B$); dans ce cas on opère dans un solvant tel que l'éther ou le tétrahydrofuranne ou encore dans le diméthylformamide à une température comprise entre 20 et 80 °C.

γ) sous forme d'imidazolide, c'est-à-dire un produit de formule générale (XIII) dans laquelle W représente un radical de formule générale (XIV) dans laquelle $R_3$ et $R_4$ forment ensemble un radical imidazolyle; dans ce cas, la réaction s'effectue dans un solvant organique tel que le tétrahydrofuranne ou le diméthylformamide ou un mélange de ces solvants à une température comprise entre 20 et 80 °C.

Pour obtenir les produits de formule générale (XIII) définis comme précédemment en A et dans laquelle W représente un radical acétyle, on fait réagir le dérivé éthoxymagnésien du malonate d'éthyle sur un halogénure de l'acide de formule générale ($I_B$) défini comme précédemment.

On opère généralement dans un solvent organique tel qu'un éther ou un alcool ou un mélange de ces solvants, en présence d'un accepteur d'acide tel que la triéthylamine à une température comprise entre 10 °C la température de reflux du mélange réactionnel.

Pour obtenir les produits de formule générale (XIII) définis comme précédemment en A et dans laquelle W représente un radical de formule générale (XV) défini comme précédemment, on fait réagir une hydrazine de formule générale:

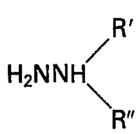

(XIX)

dans laquelle R' et R'' représente des radicaux alcoyle identiques ou différents sur un produit de formule générale (I) dans laquelle Y représente un radical de formule générale (II) défini comme précédemment, $R_1$ représente un atome d'hydrogène, R représente un radical pyridyle-3, m est égal à 1 et n est égal à 0, c'est-à-dire un produit de formule générale:

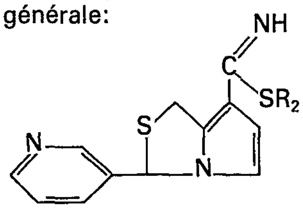

($I_C$)

On opère généralement dans un solvant organique tel que l'éthanol, à une température comprise entre 20 et 80 °C.

Les produits de formule générale ($I_C$) peuvent être préparés par action d'un produit de formule générale (XI) défini comme précédemment sur un produit de formule générale (XII) dans laquelle les symboles sont définis comme précédemment en a).

On opère généralement dans un solvant organique tel que l'acétone ou un mélange d'acétone et de diméthylformamide, à une température comprise entre 0 et 50 °C.

Pour obtenir les produits de formule générale (XIII) définis précédemment en B et dans laquelle le radical de formule générale (XVI) est défini comme en $a_2$) à l'exception pour Q de représenter un atome de soufre ou un radical imino, on fait réagir l'ammoniac ou une amine de formule générale:

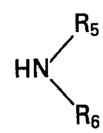

(XX)

dans laquelle $R_5$ et $R_6$ sont définis comme précédemment sur un produit de formule générale (I) dans laquelle Y représente un radical carboxy et les autres symboles sont définis comme précédemment en b), c'est-à-dire un produit de formule générale:

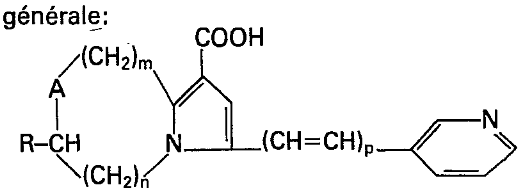

($I_D$)

Il est particulièrement avantageux d'utiliser l'acide de formule générale ($I_D$) sous une forme activée telle que le chlorure d'acide ou de le faire réagir avec le N,N'-carbonyl-diimidazole ou un chloroformiate d'alcoyle avant de faire réagir l'ammoniac ou l'amine de formule générale (XX).

Généralement il est préférable de faire réagir le chlorure d'acide et d'effectuer la réaction dans un solvant organique tel que le chloroforme ou le chlorure de méthylène à une température comprise entre 0 °C et le reflux du mélange réactionnel.

Les acides de formule générale ($I_D$) peuvent être préparés par hydrolyse des nitriles de formule générale ($I_A$) définis comme précédemment.

L'hydrolyse peut être effectuée par toute méthode connue de l'homme du métier pour transformer un nitrile en acide sans toucher au reste de la molécule. Il est généralement avantageux d'effectuer l'hydrolyse en milieu basique dans un alcool à haut point d'ébullition, par exemple au moyen de potasse dans l'éthylèneglycol entre 100 °C et la température de reflux du mélange réactionnel.

Les nitriles de formule générale ($I_A$) peuvent être obtenus par action d'un produit de formule générale (IV) sur un produit de formule générale (V) comme il a été dit précédemment.

Pour obtenir les produits de formule générale (XIII) définis comme précédemment en B et dans laquelle le radical de formule générale (XVI) est tel que Q représente un atome d'oxygène et M représente un radical de formule générale (XVII) dans laquelle $R_5$ et $R_6$ représente un atome d'hydrogène, on hydrolyse un nitrile de formule générale ($I_A$) défini comme précédemment.

L'hydrolyse peut être effectuée par tout moyen connu de l'homme du métier pour transformer un nitrile en amide sans toucher au reste de la molécule, notamment par chauffage en milieu alcalin dans un solvant organique tel que le tert-butanol à une température comprise entre 30 et 85 °C.

Pour obtenir les produits de formule générale (XIII) définis comme précédemment en B et dans laquelle le radical de formule générale (XVI) est tel que Q représente un radical imino et M est défini comme précédemment en $a_2$), on fait réagir l'ammoniac ou une amine de formule générale (XX) dans laquelle $R_5$ et $R_6$ sont définis comme précédemment sur un produit de formule générale (I) dans laquelle Y représente un radical de formule générale (II) défini comme précédemment et les autres symboles sont définis comme précédemment en b), c'est-à-dire un produit de formule générale:

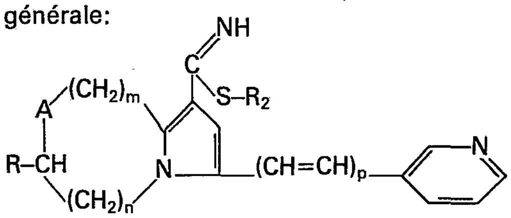
($I_E$)

La réaction s'effectue généralement dans un solvant organique tel que le chloroforme en présence d'un acide faible tel que l'acide acétique à une température comprise entre 20 et 65 °C.

Le produit de formule générale ($I_E$) peut être préparé par action d'un produit de formule générale (XI) sur un produit de formule générale (XII) dans laquelle les symboles sont définis comme précédemment en b).

On opère généralement dans un solvant organique tel que l'acétone ou un mélange d'acétone et de diméthylformamide, à une température comprise entre 0 et 50 °C.

Pour obtenir les produits de formule générale (XIII) définis comme précédemment en B et dans laquelle le radical de formule générale (XVI) est tel que Q représente un atome de soufre et M représente un radical de formule générale (XVI) dans laquelle $R_5$ et $R_6$ représente un atome d'hydrogène et p est égal à 0, on transforme un nitrile de formule générale ($I_A$) défini comme précédemment en thioamide sans toucher au reste de la molécule. Il est particulièrement avantageux de faire agir sur le nitrile de formule générale ($I_A$) le sulfure d'hydrogène dans un solvant tel que la pyridine en présence de triéthylamine, en opérant à une température comprise entre 0 et 50 °C.

Pour obtenir les produits de formule générale (XIII) définis comme précédemment en B et dans laquelle le radical de formule générale (XVII) est tel que défini précédemment en $b_2$), on fait agir une dialcoylhydrazine de formule générale (XIX) définie comme précédemment sur un produit de formule générale ($I_E$) défini comme précédemment.

On opère généralement dans un solvant organique tel que l'éthanol à une température comprise entre 20 et 80 °C.

Pour obtenir les produits de formule générale (XIII) définis comme précédemment en B et dans laquelle le radical de formule générale (XVI) est tel que Q représente un atome d'oxygène et M représente un radical de fomule générale (XVII) dans laquelle $R_5$ représente un atome d'hydrogène et $R_6$ représente un radical alcoyle contenant 1 à 5 atomes de carbone substitué par un radical hydroxyalcoylamino, étant entendu que le radical alcoyle et la portion alcoyle du radical hydroxyalcoylamino contiennent le même nombre d'atomes de carbone, on fait agir un aminoalcool de formule générale:

$$H_2N\text{–}G\text{–}OH \qquad (XXI)$$

dans laquelle G représente un radical alcoylène contenant 1 à 5 atomes de carbone, sur un nitrile de formule générale ($I_A$) défini comme précédemment.

On opère généralement dans un excès d'aminoalcool de formule générale (XXI) en présence de chlorure de lithium et à une température comprise entre 100 °C et la température de reflux du mélange réactionnel.

Pour obtenir les produits de formule générale (XIII) définis comme précédemment en B et dans laquelle le radical de formule général (XVI) est défini comme précédemment en $c_2$), on fait agir un produit de formule générale:

$$H_2NCH_2CH_2\text{–}T\text{–}H \qquad (XXII)$$

dans laquelle T représente un atome de soufre ou un radical amino, sur un nitrile de formule générale ($I_A$) défini comme précédemment.

On opère généralement dans un solvant organique tel qu'un alcool ou dans un excès de produit de formule générale (XXII) à une température comprise entre 60 °C et la température de reflux du mélange réactionnel.

Les nouveaux produits de formule générale (I) ainsi que les produits thérapeutiquement actifs de formule générale (XIII) peuvent être purifiés par les méthodes connues habituelles, par exemple cristallisation, chromatographie ou extractions successives en milieu acide et basiques.

Les nouveaux produits de formule générale (I) ainsi que les produits thérapeutiquement actifs de formule générale (XIII) peuvent être transformés en sel d'addition avec les acides par action d'un acide dans un solvant organique tel qu'un alcool, une cétone, un éther ou un solvant chloré. Le sel formé précipite, éventuellement après concentration de sa solution; il est séparé par filtration ou décantation.

Les nouveaux produits de formule générale (I) dans laquelle Y représente un radical carboxy, ainsi que les produits thérapeutiquement actifs de formule générale (XIII) contenant dans leur molécule un groupement acide peuvent être transformés en sels métalliques ou en sels d'addition avec les bases azotées, par toute méthode connue de l'homme du métier pour effectuer cette salification sans toucher au reste de la molécule.

Les produits thérapeutiquement actifs de formule générale (XIII) définis comme précédemment en A sont décrits dans la demande EP-A-115979; ils présentent des propriétés pharmacologiques intéressantes associées à une faible toxicité. Ils se sont montrés actifs à des concentrations inférieures à 50 mg/l dans le test de mesure de l'activité inhibitrice in vitro vis-à-vis de l'agrégation plaquettaire induite par l'O-octadécyl-1 O-acétyl-2 ns-glycérophosphorylcholine-3 (P. A. F.-Acéther) selon la technique de G. V. R. Born et coll., J. Physiol., 168, 178 (1963).

Leur dose toxique (exprimée par la $DL_{50}$) chez la souris est généralement comprise entre 300 et 900 mg/kg par voie orale.

Ces propriétés permettent d'envisager un traitement des affections allergiques et inflammatoires et d'une façon générale des affections dans lesquelles le rôle physiopathologique du PAF-Acéther peut être incriminé.

Les produits thérapeutiquement actifs de formule générale (XIII) définis comme précédemment en B sont décrits dans la demande EP-A-118321; ils présentent d'intéressantes propriétés pharmacologiques les rendant utiles dans le traitement prophylactique et thérapeutique des affections thrombotiques. Ils se sont montrés actifs à des concentrations inférieures à 50 mg/l dans le test de mesure de l'activité inhibitrice in vitro vis-à-vis de l'agrégation plaquettaire induite par le collagène selon la technique de born G. V. R. et coll., [J. Physiol., 168, 178 (1963)].

Leur $DL_{50}$ est généralement comprise entre 300 et 900 mg/kg, par voie orale chez la souris.

Pour l'emploi médicinal, il peut être fait usage des produits thérapeutiquement actifs de formule générale (XIII) tels quels ou à l'état de sels pharmaceutiquement acceptables, c'est-à-dire non toxiques aux doses d'utilisation.

Comme exemples de sels pharmaceutiquement acceptables on peut citer les sels d'addition avec les acides minéraux tels que chlorhydrates, sulfates, nitrates, phosphates ou organiques tels que acétates, propionates, succinates, benzoats, fumarates, maléates, méthanesulfonates, iséthionates, théophylline-acétates, salicylates, phénolphtalinates, méthylène-bis-β-oxynaphtoates ou des dérivés de substitution de ces composés. Lorsqu'ils peuvent exister, on peut encore citer les sels avec les métaux alcalins tels que les sels de sodium, potassium ou lithium, avec les métaux alcalino-terreux tels que les sels de calcium ou de magnésium, et les sels d'addition avec les bases organiques tels que les sels d'éthanolamine ou de lysine.

Comme sels en lesquels on peut transformer les produits de formule générale (I), on peut citer les sels thérapeutiquement acceptables cités ci-dessus pour les produits de formule générale (XIII).

Les exemples suivants, donnés à titre non limitatif, montrent comme l'invention peut être mise en pratique. Les exemples d'application, donnés également à titre non limitatif, montrent comment on peut mettre en pratique les produits de l'invention pour les transformer en produits de formule générale (XIII) thérapeutiquement actifs.

Exemple 1

48 g de (pyridyl-3)-3 1H,3H-pyrrolo[1,2-c] thiazolecarbonitrile-7 sont ajoutés à une solution de 41,7 g de potasse en pastilles dans 400 $cm^3$ d'éthylèneglycol. Le mélange réactionnel est chauffé à une température voisine de 150 °C pendant 6 heures et 30 minutes. Après 16 heures d'agitation à une température voisine de 20 °C, on évapore le solvant sous pression réduite (5 mm de mercure; 0,7 kPa) à une température voisine de 100 °C. Le résidu est dissous dans 380 $cm^3$ d'eau distillée. La solution obtenue est additionnée de 0,5 g de noir décolorant, filtrée et amenée à un pH voisin de 4 par addition d'une solution aqueuse concentrée d'acide chlorhydrique en maintenant la température voisine de 20 °C. Les cristaux apparus sont séparés par filtration, lavés 3 fois avec 600 $cm^3$ au total d'eau distillée puis séchés sous pression réduite (20 mm de mercure; 2,7 kPa) à une température voisine de 20 °C en présence de potasse en pastilles. On obtient ainsi 49,5 g de produit brut fondant à 177 °C. Ce produit est dissous dans 840 $cm^3$ d'éthanol bouillant. La solution obtenue est additionnée de 0,5 g de noir décolorant puis filtrée à chaud; li filtrat est refroidi à une température voisine de 4 °C pendant 1 heure. Les cristaux apparus sont séparés par filtration, lavés 3 fois avec 45 $cm^3$ au total d'éthanol refroidi à une température voisine de 4 °C puis 3 fois avec 90 $cm^3$ au total d'éther diéthylique et séchés sous pression réduite (20 mm de mercure; 2,7 kPa) à une température voisine de 20 °C en présence de potasse en pastilles. On obtient ainsi 36,6 g d'acide (pyridyl-3)-3 1H,3H-pyrrolo[1,2-c] thiazolecarboxylique-7 sous forme de cristaux crème fondant à 178 °C.

Le (pyridyl-3)-3 1H,3H-pyrrolo[1,2-c] thiazolecarbonitrile-7 peut être obtenu de la façon suivante:

Un mélange de 249,4 g d-acide formyl-3 (pyridyl-3)-2 thiazolidinecarbocylique-4, de 457 g de chloro-2 acrylonitrile et de 0,2 g d'hydroquinone dans 1760 $cm^3$ d'anhydride acétique est chauffé à une température comprise entre 110 °C et 117 °C pendant 70 minutes. Le solvant est ensuite évaporé sous pression réduit (20 mm de mercure; 2,7 kPa) à une température comprise entre 50 °C et 80 °C. Le résidu est repris par 400 $cm^3$ d'eau distil-

lée; la suspension obtenue est amenée à un pH voisin de 10 par addition d'une solution aqueuse de soude 5 N puis extraite 4 fois par 2500 cm$^3$ au total de chlorure de méthylène. Les extraits organiques sont réunis, lavés 3 fois par 1500 cm$^3$ au total d'eau distillée, séchés sur du sulfate de magnésium anyhdre, additionnés de 0,5 g de noir décolorant, filtrés et concentrés à ces sous pression réduite (20 mm de mercure; 2,7 kPa) à une température voisine de 40 °C.

Le résidu obtenu est dissous dans un mélange de 250 cm$^3$ d'acétate d'éthyle et de 500 cm$^3$ d'une solution aqueuse d'acide chlorhydrique 1,8 N. La phase organique est séparée par décantation et extraite 2 fois par 200 cm$^3$ au total d'eau distillée. Les extraits aqueux sont réunis, lavés 5 fois par 500 cm$^3$ au total d'acétate d'éthyle, additionnés de 0,5 g de noir décolorant et filtrés; le filtrat est amené à un pH voisin de 10 par addition d'une solution aqueuse de soude 10 N à une température voisine de 4 °C puis extrait 3 fois avec 650 cm$^3$ au total d'acétate d'éthyle. Les extraits organiques sont réunis, lavés 3 fois par 450 cm$^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 40 °C. On obtient ainsi 71,2 g de produit brut. Ce produit est dissous dans 150 cm$^3$ de propanol-2 bouillant et la solution obtenue est additionnée de 0,5 g de noir décolorant puis filtrée à chaud; le filtrat est refroidi à une température voisine de 4 °C pendant 1 heure. Les cristaux apparus sont séparés par filtration, lavés 3 fois par 30 cm$^3$ au total de propanol-2 refroidi à une température voisine de 4 °C, 3 fois par 60m$^3$ au total d'oxyde d'isopropyle puis séchés sous pression réduite (20 mm de mercure; 2,7 kPa) à une température voisine de 20 °C en présence de potasse en pastilles. On obtient ainsi 44 g de (pyridyl-3)-3 1H,3H-pyrrolo[1,2-c] thiazolecarbonitrile-7 sous forme de cristaux de couleur ocre fondant à 117 °C.

L'acide formyl-3 (pyridyl-3)-2 thiazolidinecarboxylique-4 peut être obtenu de la façon suivante:

A 1200 cm$^3$ d'acide formique, on ajoute 250 g d'acide (pyridyl-3)-2 thiazolidinecarboxylique-4 en maintenant la température du milieu réactionnel inférieure à 25 °C. A la solution ainsi obtenue on ajoute, en 1 heure, 875 g d'anhydride acétique en maintenant la température entre 10 °C et 18 °C.

Après 20 heures d'agitation, à une température voisine de 20 °C, on évapore le solvant sous pression réduite (20 mm de mercure; 2,7 kPa) à une température voisine de 60 °C, reprend le résidu avec 1000 cm$^3$ d'éthanol, chauffe à ébullition pendant 5 minutes puis refroidit à une température voisine de 4 °C pendant 1 heure; les cristaux apparus sont séparés par filtration, lavés 3 fois par 600 cm$^3$ au total d'éthanol puis 3 fois par 300 cm$^3$ au total d'éther diéthylique et séchés sous pression réduite (20 mm de mercure; 2,7 kPa) à une température voisine de 20 °C en présence de potasse en pastilles. On obtient ainsi 234,5 g d-acide formyl-3 (pyridyl-3)-2 thiazolidinecarboxylique-4 sous forme de cristaux crème fondant à 214 °C.

L'acide (pyridyl-3)-2 thayzolinecarboxylique-4 peut être préparé selon A. Banashek et M. I.Shchukina, J. Gen. chem. U.S.S.R., 31, 1374 (1961); Chem. Abstr. 55, 24739 h, (1961).

Exemple 2

Une suspension de 7 g de (pyridyl-3)-3 1H,3H-pyrrolo [1,2-c] thiazolecarbothioamide-7 et de 4,2 g d'iodure de méthyle dans 250 cm$^3$ d'acétone est agitée à une température voisine de 20 °C pendant 16 heures. On ajoute alors à la suspension 50 cm$^3$ de diméthylformamide et on poursuit l'agitation pendant 3 jours supplémentaires. Les cristaux sont alors séparés par filtration, lavés 3 fois par 90 cm$^3$ au total d'acétone et séchés sous pression réduite (20 mm de mercure; 2,7 kPa) à une température voisine de 20 °C en présence de potasse en pastilles. On obtient ainsi 9,5 g d'iodhydrate de (pyridyl-3)-3 1H,3H-pyrrolo [1,2-c] thiazolethiocarboximidate-7 de S-méthyle sous forme de cristaux jaunes fondant à 193–194 °C.

Le (pyridyl-3)-3 1H,3H-pyrrolo [1,2-c] thiazolecarbothioamide-7 est préparé de la façon suivante:

8,1 g de (pyridyl-3)-3 1H,3H-pyrrolo [1,2-c] thiazolecarboyamide-7 sont ajoutés à 100 cm$^3$ de pyridine saturée à une température voisine de 20 °C par un courant gazeux de sulfure d'hydrogène. La suspension obtenue est additonnée de 7,3 g de pentasulfure de phosphore puis chauffée à l'ébullition pendant 2 heures sous courant gazeux de sulfure d'hydrogène. La solution obtenue est refroidie à une température voisine de 20 °C puis est versée sur 1200 cm$^3$ d'eau distillée. La suspension obtenue est conservée à une température voisine de 4 °C pendant 16 heures. Les cristaux sont séparés par filtration, lavés 5 fois par 500 cm$^3$ au total d'eau distillée, 5 fois par 25 cm$^3$ au total d'éthanol et 5 fois par l'éther diéthylique puis séchés sous pression réduite (20 mm de mercure; 2,7 kPa) à une température voisine de 20 °C en présence de potasse en pastilles. On obtient ainsi 7,7 g de produit brut fondant à 205 °C. Le produit est dissous dans 180 cm$^3$ de butanol-1 bouillant. La solution obtenue est refroidie à une température voisine de 4 °C pendant 1 heure. Les cristaux apparus sont séparés par filtration, lavés 3 fois par 15 cm$^3$ au total de butanol-1 refroidi à une température voisine de 4 °C et 3 fois par 60 cm$^3$ au total d'éther diéthylique puis séchés sous pression réduite (20 mm de mercure; 2,7 kPa) à une température voisine de 20 °C en présence de potasse en pastilles. On obtient ainsi 7,1 g de (pyridyl-3)-3 1H,3H-pyrrolo [1,2-c] thiazolecarbothioamide-7 sous forme de cristaux crème fondant à 205 °C.

Le (pyridyl-3)-3 1H,3H-pyrrolo [1,2-c] thiazolecarboxamide-7 est préparé de la façon suivante:

On chauffe sous agitation pendant 3 heures et 15 minutes à une température voisine de 85 °C un mélange de 13,6 g de (pyridyl-3)-3 1H,3H-pyrrolo [1,2-c] thiazolecarbonitrile-7 et de 21 g de potasse en poudre dans 150 cm$^3$ d'alcool tert-butylique. On évapore ensuite le solvant sous pression réduite (20 mm de mercure; 2,7 kPa) à une température voisine de 40 °C puis remet le résidu en suspension dans 500 $cm^3$ d'eau distillée et agite pendant 5 minutes à une température voisine de 20 °C; les cristaux apparus sont séparés par filtration, lavés 5 fois par 500 $cm^3$ au total d'eau distillée, 3 fois par 60 $cm^3$ au total d'éthanol, puis 3 fois par 60 $cm^3$ au total d'éther diéthylique et séchés sous pression réduite (20 mm de mercure; 2,7 kPa) à une température voisine de 20 °C en présence de potasse en pastilles. On obtient ainsi 12,1 g de produit brut fondant à 208 °C. Ce produit, réuni avec 2,2 g de produit préparé de la même manière dans une autre opération antérieure est dissous dans 800 $cm^3$ d'éthanol bouillant. La solution obtenue est additionnée de 0,5 g de noir décolorant puis filtrée à chaud. Le filtrat est refroidi pendant 1 heure à une température voisine de 4 °C; les cristaux apparus sont séparés par filtration, lavés 3 fois par 30 $cm^3$ au total d'éthanol refroidi à une température voisine de 4 °C et séchés sous pression réduite (20 mm de mercure; 2,7 kPa) à une température voisine de 20 °C en présence de potasse en pastilles. On obtient ainsi 10,7 g de (pyridyl-3)-3 1H,3H-pyrrolo [1,2-c] thiazoloecarboxamide-7 sous forme de cristaux blancs fondant à 210 °C.

Le (pyridyl-3)-2 1H,3H-pyrrolo [1,2-c] thiazolecarbonitrile-7 est préparé comme à l'exemple 1.

Exemple 3

Une suspension de 403 g d'acide N-nicotinoyl-thiazolidine-carboxylique-4 dans un mélange de 1350 $cm^3$ de chloro-2 acrylonitrile et de 1750 $cm^3$ d'anhydride acétique est chauffée à 90 °C pendant 2 heures 40 minutes. Durant cette période, on observe un passage par une phase homogène limpide au bout de 30 minutes, suivi d'une précipitation 10 minutes plus tard. Après refroidissement à une température voisine de 4 °C pendant 16 heures, les cristaux apparus sont séparés par filtration, lavés 2 fois par 200 $cm^3$ au total d'anhydride acétique, 3 fois par 300 $cm^3$ au total d'acétone et séchés sous pression réduite (20 mm de mercure; 2,7 kPa) à une température voisine de 20 °C en présence de potasse e pastilles. Le produit ainsi obtenu est mis en suspension dans 2400 $cm^3$ d'une solution aqueuse de soude 2 N. Après agitation à une température voisine de 20 °C pendant 1 heure et 30 minutes, les cristaux apparus sont séparés par filtration, lavés 5 fois par 1250 $cm^3$ au total d'eau distillée, 3 fois par 1200 $cm^3$ au total d'éthanol, 3 fois par 900 $cm^3$ au total d'éther diéthylique et séchés sous pression réduite (20 mm de mercure; 2,7 kPa) à une température voisine de 20 °C en présence de potasse en pastilles. On obtient ainsi 159,7 g de (pyridyl-3)-5 1H,3H-pyrrolo[1,2-c] thiazolecarbonitrile-7 sous forme de cristaux crème fondant à 170 °C.

L'acide N-nicotinoyl thiazolidinecarboxylique-4 peut être obtenu de la façon suivante:

A une solution de 400 g d'acide thiazolidinecarboxylique-4 et de 613 g de triéthylamine dans 4500 $cm^3$ de chloroforme, on ajoute, en 1 heure à une température comprise entre 30 et 52 °C, 534 g de chlorhydrate de chlorure de nicotinoyle. La solution obtenue est chauffée à une température voisine de 64 °C pendant 4 heures. Après agitation à une température voisine de 20 °C pendant 16 heures, les cristaux apparus sont séparés par filtration, lavés 3 fois par 1500 $cm^3$ au total de chloroform puis 3 fois par 1500 $cm^3$ au total d'éther diéthylique et séchés sous pression réduite (20 mm de mercure; 2,7 kPa) à une température voisine de 20 °C en présence de potasse en pastilles. On obtient ainsi 403 g d'acide N-nicotinoyl thiazolidinecarboxylique-4 sous forme de cristaux blancs fondant à 190 °C.

Exemple 4

Une suspension de 44 g de N-nicotinoyl L-proline dans un mélange de 160 $cm^3$ de chloro-2 acrylonitrile et de 200 $cm^3$ d'anhydride acétique est chauffée progressivement à 90 °C. Après dissolution des réactifs dans le milieu réactionnel, on observe une précipitation donnant naissance à une suspension. On poursuit le chauffage de la suspension pendant 3 heures et 30 minutes à une température voisine de 90 °C. Après refroidissement à une température voisine de 4 °C pendant 1 heure, les cristaux apparus sont séparés par filtration, lavés 2 fois par 50 $cm^3$ au total d'anhydride acétique, 3 fois par 300 $cm^3$ au total d'acétone et séchés sous pression réduite (20 mm de mercure; 2,7 kPa) à une température voisine de 20 °C en présence de potasse en pastilles. Le produit ainsi obtenu est repris par 500 $cm^3$ d'une solution aqueuse de soude 1 N. L'huile apparue est dissoute dans 250 $cm^3$ d'acétate d'éthyle. La phase organique est séparée par décantation et la phase aqueuse est extraite 3 fois par 750 $cm^3$ au total d'acétate d'ethyle. Les extraits organiques sont réunis, lavés 3 fois par 750 $cm^3$ au total d'eau distillée, séchés sur du carbonate de potassium anhydre, additionnés de 1 g de noir décolorant, filtrés et concentrés à sec sous pression réduite (20 mm de mercure; 2,7 kPa) à une température voisine de 60 °C. On obtient ainsi 25,2 g de produit brut. Ce produit est chromatographié sur une colonne de 4 cm de diamètre contenant 250 g de silice (0,063–0,2 mm) en éluant par de l'acétate d'éthyle et en recueillant des fractions de 400 $cm^3$. Les deux premières fractions sont éliminées, les trois fractions suivantes sont réunies et concentrées à sec sous pression réduite (20 mm de mercure; 2,7 kPa) à une température voisine de 60 °C. On obtient ainsi 21 g de produit. Ce produit est dissous dans 100 $cm^3$ d'éthanol bouillant. La solution obtenue est refroidie à une température voisine de 4 °C pendant 1 heure. Les cristaux apparus sont séparés par filtration, lavés 3 fois par 30 $cm^3$ au total d'éthanol refroidi à une température voisine de 4 °C puis 3 fois par 150 $cm^3$ au total d'oxyde d'isopropyle. On obtient ainsi 16,1 g de (pyridyl-3)-5 dihydro-2,3 1H,3H-pyrrolizinecarbonitrile-7 sous forme de cristaux crème fondant à 112 °C.

La N-nicotinoyl L-proline peut être préparée selon F. Coustou et B. Bellegarde, brevet ouest-allemand 2537590.

Exemple 5

Une suspension de 13,2 g d'acide N-[(pyridyl-3)-3 acryloyl] thiazolidinecarboxylique-4 dans un mélange de 39,6 $cm^3$ de chloro-2 acrylonitrile et de 52 $cm^3$ d'anhydride acétique est chauffée à 83 °C environ pendant 4 heures. Après refroidissement pendant 16 heures à une température voisine de 4 °C, les cristaux apparus sont séparés par filtration, lavés deux fois par 10 $cm^3$ au total d'anhydride acétique et trois fois par 60 $cm^3$ au total d'acétone. Le produit ainsi obtenu est mis en suspension dans 70 $cm^3$ d'eau distillée. Le mélange est amené à un pH voisin de 10 par addition d'une solution aqueuse de soude 2 N. Après agitation à une température voisine de 20 °C pendant une heure, les cristaux apparus sont séparés par filtration, lavés trois fois par 60 $cm^3$ au total d'eau distillée, deux fois par 40 $cm^3$ au total d'acétone, deux fois par 40 $cm^3$ au total d'éther diéthylique et séchés sous pression réduite (20 mm de mercure; 2,7 kPa) à une température voisine de 20 °C en présence de potasse en pastilles. On obtient ainsi un mélange de cyano-6 et cyano-7 [(pyridyl-3)-2 vinyl]-5 1H,3H-pyrrolo[1,2-c] thiazole sous forme de cristaux beige fondant à 170 °C.

L'acide N-[(pyridyl-3)-3 acryloyl] thiazolecarboxylique-4 peut être obtenu de la façon suivante:

A une solution de 22,5 g d'acide thiazolidinecarboxylique-4 dans un mélange de 47 $cm^3$ de triéthylamine et de 250 $m^3$ de chloroforme, on ajoute, en 30 minutes à une température comprise entre 20 °C et 35 °C, 34,1 g de chlorhydrate de chlorure de (pyridyl-3)-3 acryloyle. Le mélange réactionnel est chauffé au reflux pendant 16 heures, puis concentré à sec sous pression réduite (20 mm de mercure; 2,7 kPa). Le résidu obtenu, additionné de 500 $cm^3$ d'eau distillée, est porté au reflux. Après addition de 1 g de noir décolorant, le mélange est filtré à chaud et le filtrat refroidi à 4 °C pendant 16 heures. Les cristaux apparus sont séparés par filtration, lavés deux fois par 100 $cm^3$ au total d'eau distillée, et une fois par 30 $cm^3$ d'éthanol puis séchés sous pression réduite (20 mm de mercure; 2,7 kPa) en présence de potasse en pastilles à une température voisine de 20 °C. On obtient ainsi 21,1 g de produit brut fondant à 173 °C. Ce produit est dissous dans 400 $m^3$ d'éthanol bouillant. La solution obtenue est additionnée de 1 g de noir décolorant et filtrée à chaud. Le filtrat est refroidi à une température voisine de 4 °C pendant 16 heures. Les cristaux apparus sont séparés par filtration, lavés deux fois par 40 $cm^3$ au total d'éthanol et séchés sous pression réduite (20 mm de mercure; 2,7 kPa) à une température voisine de 20 °C en présence de potasse en pastilles. On obtient ainsi 13,5 g d'acide N-[(pyridyl-3)-3 acryloyl] thiazolidinecarboxylique-4 fondant à 176 °C.

Le chlorhydrate du chlorure de (pyridyl-3)-3 acryloyle peut être obtenu de la façon suivante:

200 $cm^3$ de chlorure de thionyle sont ajoutés en 15 minutes à 50 g d'acide (pyridyl-3)-3 acrylique. Le mélange réactionnel est ensuite chauffé au reflux pendant 5 heures. L'excès de chlorure de thionyle est distillé puis le mélange réactionnel est concentré à sec après addition de 300 $cm^3$ de cyclohexane anhydre. Cette dernière opération est répétée une fois. Le résidus obtenu est additionné de 200 $cm^3$ de chloroforme et chauffé aux reflux pendant 15 minutes. Après refroidissement, les cristaux sont séparés par filtration, lavés une fois par 50 $cm^3$ de chloroforme et deux fois par 200 $cm^3$ au total d'hexane, puis séchés sous pression réduite (20 mm de mercure; 2,7 kPa) en présence de potasse en pastilles à une température voisine de 20 °C. On obtient ainsi 55 g de chlorhydrate du chlorure de (pyridyl-3)-3 acryloyle fondant à 187 °C.

L'acide (pyridyl-3)-3 acrylique peut être préparé selon L. Panizzon, Helv. Chim. Acta, 24, 24E (1941).

Exemple 6

Une suspension de 36,3 g d'acide N-nicotinoyl méthyl-2 thiazolidinecarboxylique-4 dans un mélange de 115 $cm^3$ de chloro-2 acrylonitrile et de 200 $cm^3$ d'anhydride acétique est chauffée à une température voisine de 90 °C pendant 3 heures. La solution obtenue est concentrée à sec sous pression réduite (20 mm de mercure; 2,7 kPa) à une température voisine de 70 °C et le résidu obtenu est repris par 200 $cm^3$ d'eau distillée. La suspension obtenue est amenée à un pH voisin de 10 par addition de 80 $cm^3$ d'une solution aqueuse de soude 10 N en maintenant le mélange réactionnel à une température voisine de 20 °C. La suspension obtenue est additionnée de 250 $cm^3$ de chlorure de méthylène et agitée à une température voisine de 20 °C pendant 16 heures. La phase organique est séparée par décantation et la phase aqueuse est extraite 5 fois par 500 $cm^3$ au total de chlorure de méthylène. Les extraits organiques sont réunis, lavés 5 fois par 500 $cm^3$ au total d'eau distillée et 5 fois par 400 $cm^3$ au total d'une solution aqueuse d'acide chlorhydrique 2 N. La phase aqueuse est séparée par décantation, additionnée de 0,5 g de noir décolorant, filtrée, amenée à un pH voisin de 10 par addition d'une solution aqueuse de soude 10 N et extraite 5 fois par 500 $cm^3$ au total de chlorure de méthylène. Les extraits organiques sont réunis, séchés sur du sulfate de magnésium anhydre, additionnés de 0,5 g de noir décolorant, filtrés et concentrés à sec sous pression réduite (20 mm de mercure; 2,7 kPa) à une température voisine de 50 °C. On obtient ainsi 19,3 g de produit bruit. Ce produit est chromatographié sur une colonne de 6 cm de diamètre contenant 480 g de silice (0,04–0,063 mm). On élue par un mélange de cyclohexane et d'acétate d'éthyle (50–50 en volumes) sous une pression de 0,5 bar (51 kPa) en recueillant des fractions de 200 $cm^3$. Les 8 premières fractions sont éliminées, les 8 fractions suivantes sont réunies, concentrées à sec sous pression réduite (20 mm de mercure; 2,7 kPa) à une température voisine de 50 °C. On obtient ainsi 17,6 g de mélange de cyano-6 et de cyano-7 méthyl-3 (pyridyl-3)-5 1H,3H-pyrrolo[1,2-c] thiazole sous forme d'une huile jaune dans un rapport 50–50 (d'après le spectre de RMN).

[Rf = 0,35 et 0,4; chromatographie sur couche mince de gel de silice; solvant: cyclohexane-acétate d'éthyle (50–50 en volumes).]

L'acide N-nicotinoyl méthyl-2 thiazolidinecarboxylique-4 peut être préparé de la façon suivante:

A une suspension de 44,1 g d'acide méthyl-2 thiazolidinecarboxylique-4 et de 61,8 g de triéthylamine dans 500 $cm^3$ de chloroforme, on ajoute en 25 minutes, à une température comprise entre 20 °C et 47 °C, 53,4 g de chlorhydrate de chlorure de nicotinoyle. La suspension obtenue est chauffée à une température voisine de 65 °C pendant 1 heure et 45 minutes puis la solution obtenue est agitée à une température voisine de 20 °C pendant 16 heures. Le solvant est évaporé sous pression réduite (20 mm de mercure; 2,7 kPa) à une température voisine de 50 °C et le résidu obtenu est mis en suspension dans 300 $cm^3$ d'acétone. Après 2 heures d'agitation à une température voisine de 20 °C, les cristaux apparus sont séparés par filtration, lavés 2 fois par 400 $cm^3$ au total d'acétone et séchés sous pression réduite (20 mm de mercure; 2,7 kPa) à une température voisine de 20 °C en présence de potasse en pastilles. Le solide obtenu est mis en suspension dans 250 $cm^3$ d'eau distillée et les cristaux apparus sont séparés par filtration, lavés 3 fois par 450 $cm^3$ au total d'eau distillée et 3 fois par 60 $cm^3$ au total d'acétone et séchés sous pression réduite (20 mm de mercure; 2,7 kPa) à une température voisine de 20 °C en présence de potasse en pastilles. On obtient ainsi 36,5 g d'acide N-nicotinoyl méthyl-2 thiazolidinecarboxylique-4 sous forme de cristaux crème fondant à 190 °C.

L'acide méthyl-2 thiazolidinecarboxylique-4 peut être préparé selon H. T. Nagasawa, D. J. W. Goon, R. T. Zera et D. L. Yuzon, J. Med. Chem., 25, 489 (1982).

Exemple 7

Une suspension de 45,2 g d'acide N-nicotinoyl phényl-2 thiazolidinecarboxylique-4 dans un mélange de 115 $cm^3$ de chloro-2-acrylonitrile et de 180 $cm^3$ d'anhydride acétique est chauffée à une température voisine de 90 °C pendant 3 heures et la solution obtenue est agitée à une température voisine de 20 °C pendant 16 heures. Le solvant est évaporé sous pression réduite (20 mm de mercure; 2,7 kPa) à une température voisine de 60 °C et le résidu est repris à une température voisine de 4 °C par un mélange de 300 $cm^3$ d'eau distillée, de 400 $cm^3$ d'une solution aqueuse de soude 10 N et de 500 $cm^3$ d'acétate d'éthyle. La phase organique est séparée par décantation et la phase aqueuse est extraite 3 fois par 1500 $cm^3$ au total d'acétate d'éthyle et 4 fois par 2000 $cm^3$ au total de chlorure de méthylène. Les extraits organiques sont réunis, lavés 4 fois par 1000 $cm^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 60 °C. On obtient ainsi 33,8 g de produit brut. Ce produit est chromatographié sur une colonne de 6 cm de diamètre contenant 480 g de silice (0,04–0,063 mm). On élue par une mélange de cyclohexane et d'acétate d'éthyle (50–50 en volumes) sous une pression de 0,5 bar (51 kPa) en recueillant des fractions de 100 $cm^3$. Les 17 premières fractions sont éliminées, les 14 fractions suivantes sont réunies et concentrées à sec sous pression réduite (20 mm de mercure; 2,7 kPa) à une température voisine de 60 °C. On obtient 11,3 g de mélange de cyano-6 et de cyano-7 phényl-3 (pyridyl-3)-5 1H,3H-pyrrolo [1,2-c] thiazole dans un rapport 20–80 (d'après le spectre de RMN) sous forme d'une huile brune.

[Rf = 0,35 et 0,4; chromatographie sur couche mince de gel de silice; solvant: cyclohexane-acétate d'étyle (50–50 en volumes).]

L'acide N-nicotinoyl phényl-2 thiazolidinecarboxylique-4 peut être préparé de la façon suivante:

A une solution de 94,2 g d'acide phényl-2 thiazolidinecarboxylique-4 et de 100 g triéthylamine dans 1120 $cm^3$ de chloroforme, on ajoute en 20 minutes, à une température comprise entre 32 et 54 °C, 88,1 g de chlorhydrate de chlorure de nicotinoyle. La solution obtenue est chauffée à une température voisine de 63 °C pendant 5 heures puis est agitée à une température voisine de 20 °C pendant 16 heures. Un produit cristallise. La suspension est refroidie à une température voisine de 4 °C pendant 1 heure. Les cristaux apparus sont séparés par filtration, lavés 3 fois par 300 $cm^3$ au total de chloroforme et séchés sous pression réduite (20 mm de mercure; 2,7 kPa) à une température voisine de 20 °C en présence de potasse en pastilles. On obtient ainsi 145 g de produit brut fondant à 150 °C. Ce produit est mis en suspension dans 750 $cm^3$ d'eau distillé. Les cristaux sont séparés par filtration, lavés 3 fois par 750 $cm^3$ au total d'eau distillée et séchés à l'air. On obtient ainsi 90,9 g de produit fondant à 182 °C. 15 g de ce produit sont dissous dans 200 $cm^3$ d'éthanol bouillant. La solution obtenue est additionnée de 0,5 g de noir décolorant et filtrée à chaud. Le filtrat est refroidi à une température voisine de 4 °C pendant 1 heure. Les cristaux apparus sont séparés par filtration, lavés 2 fois par 50 $cm^3$ au total d'éthanol et 3 fois par 75 $cm^3$ au total d'éther diéthylique et séchés sous pression réduite (20 mm de mercure; 2,7 kPa) à une température voisine de 20 °C en présence de potasse en pastilles. On obtient ainsi 11,4 g d'acide N-nicotinoyl phényl-2 thiazolidinecarboxylique-4 sous forme de cristaux blancs fondant à 186 °C.

L'acide phényl-2 thiazolidinecarboxylique-4 peut être préparé selon R. Riemschneider et G. A. Hoyer, Z. Naturforsch., 17 B, 765 (1962).

Exemple 8

Une suspension de 14,7 g d'acide N-nicotinoyl pipéridinecarboxylique-2 dans un mélange de 50 $cm^3$ de chloro-2 acrylonitrile et de 65 $cm^3$ d'anhydride acétique est chauffée à une température voisine de 90 °C pendant 4 heures. Après 16 heures d'agitation à une température voisine de 20 °C, les cristaux sont séparés par filtration, lavés 3 fois par 75 $cm^3$ au total d'anhydride acétique et séchés sous pression réduite (20 mm de mercure; 2,7 kPa) à une température voisine de 20 °C en

présence de potasse en pastilles. Les 10 g de produit ainsi obtenus sont dissous dans 100 $cm^3$ d'eau distillé. Après addition à une température voisine de 10 °C de 50 $cm^3$ d'une solution aqueuse de soude 10 N, on obtient une suspension que l'on agite à une température voisine de 20 °C pendant 1 heure puis extrait 3 fois par 450 $cm^3$ au total d'acétate d'éthyle. Les extraits organiques sont réunis, lavés 3 fois par 300 $cm^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 60 °C. On obtient ainsi 8,8 g de produit que l'on chromatographie sur une colonne de 6 cm de diamètre contenant 480 g de silice (0,04–0,063 mm) en éluant par de l'acétate d'éthyle sous une pression de 0,5 bar (51 kPa) et en recueillant des fractions de 100 $cm^3$. Les 14 premières fractions sont éliminées, les 6 fractions suivantes sont réunies et concentrées à sec sous pression réduite (20 mm de mercure; 2,7 kPa) à une température voisine de 60 °C. On obtient ainsi 5,2 g de (pyridyl-3)-3 tétrahydro-5,6,7,8 indolizine-carbonitrile-1 sous forme de cristaux marron fondant à 112 °C.

L'acide N-nicotinoyl pipéridinecarboxylique-2 peut être préparé de la façon suivante:

Une solution de 34,1 g de N-nicotinoyl pipéridinecarboxylate-2 d'éthyle dans un mélange de 130 $cm^3$ d'une solution aqueuse de soude 2 N et de 325 $cm^3$ d'éthanol est agitée à une température voisine de 20 °C pendant 16 heures. Le solvant est alors évaporé sous pression réduite (20 mm de mercure; 2,7 kPa) à une température voisine de 60 °C. Le résidu obtenu est dissous dans 250 $cm^3$ d'eau distillée. La solution trouble obtenue est additionnée de 0,5 g de noir décolorant et filtrée. Le filtrat, maintenu à une température de 20 °C, est amené à un pH voisin de 3 par addition de 80 °C d'une solution aqueuse d'acide chlorhydrique 4 N. La suspension obtenue est agitée à une température voisine de 20 °C pendant 1 heure et les cristaux sont séparés par filtration, lavés 4 fois par 200 $cm^3$ au total d'eau distillée, 3 fois par 150 $cm^3$ au total d'acétone et 1 fois par 50 $cm^3$ d'éther diéthylique puis séchés sous pression réduite (20 mm de mercure; 2,7 kPa) à une température voisine de 20 °C en présence de potasse en pastilles. On obtient ainsi 14,8 g d'acide N-nicotinoyl pipéridinecarboxylique-2 sous forme de cristaux blancs fondant à 194 °C.

Le N-nicotinoyl pipéridinecarboxylate-2 d'éthyle peut être préparé de la façon suivante:

A une solution de 72,9 g de pipéridinecarboxylate-2 d'éthyle dans 1120 $cm^3$ de chloroforme, on ajoute d'abord 182 g de triéthylamine à une température comprise entre 20 °C et 31 °C, puis en 35 minutes à une température comprise entre 26 °C et 50 °C, 160,2 g de chlorhydrate de chlorure de nicotinoyle. La suspension obtenue est agitée à une température voisine de 20 °C pendant 16 heures puis lavée 5 fois par 1500 $cm^3$ au total d'eau distillée, séchée sur du sulfate de magnésium anhydre, additionnée de 0,5 g de noir décolorant, filtrée et concentrée à sec sous pression réduite (20 mm de mercure; 2,7 kPa) à une température voisine de 60 °C. On obtient ainsi 174 g de produit brut que l'on chromatographie sur une colonne de 8 cm de diamètre contenant 1740 g de silice (0,063–0,2 mm) en éluant par des mélanges de cyclohexane et d'acétate d'éthyle et en recueillant des fractions de 1000 $cm^3$. Les 5 premières fractions provenant de l'élution par un mélange de cyclohexane et d'acétate d'éthyle (80–20 en volumes), les 5 fractions suivantes provenant de l'élution par un mélange de cyclohexane et d'acétate d'éthyle (70–30 en volumes), les 7 fractions suivantes provenant de l'élution par un mélange de cyclohexane et d'acétate d'éthyle (60–40 en volumes) et la fraction suivante provenant de l'élution par un mélange de cyclohexane et d'acétate d'éthyle (50–50 en volumes) sont éliminées. Les 8 fractions suivantes provenant de l'élution par un mélange de cyclohexane et d'acétate d'éthyle (50–50 en volumes) et les 7 fractions suivantes provenant de l'élution par de l'acétate d'éthyle pour sont réunies et concentrées à sec sous pression réduite (20 mm de mercure; 2,7 kPa) à une température voisine de 60 °C. On obtient ainsi 99,3 g de N-nicotinoyl pipéridinecarboxylate-2 d'éthyle sous forme d'une huile orange.

(Rf = 0,46; chromatographie sur couche mince de gel de silice; éluant: acétate d'éthyle.)

Exemple 9

On chauffe progressivement une suspension de 27,9 g d'acide N-nicotinoyl tétrahydro-2,3,5,6 thiazine-1,4 carboxylique-3 dans un mélange de 89 $cm^3$ de chloro-2 acrylonitrile et de 117 $cm^3$ d'anhydride acétique. Lorsque la température atteint 70 °C, on observe une élévation de la température jusqu'à 90 °C et une dissolution suivie, après 5 minutes à cette température, d'une cristallisation donnant naissance à une suspension. On continue le chauffage à une température voisine de 90 °C pendant 2 heures puis on refroidit le mélange réactionnel à une température voisine de 20 °C. Les cristaux sont séparés par filtration, lavés 3 fois par 75 $cm^3$ au total d'anhydride acétique puis 3 fois par 150 $cm^3$ au total d'acétone et séchés sous pression réduite (20 mm de mercure; 2,7 kPa) à une température voisine de 30 °C en présence de potasse en pastilles. On obtient ainsi 6,3 g de produit. Ce produit est mis en suspension dans 100 $cm^3$ d'eau distillée. La suspension obtenue est additionnée de 50 $cm^3$ d'une solution aqueuse de soude 5 N puis extraite 3 fois par 300 $cm^3$ au total d'acétate d'éthyle. Les extraits organiques sont réunis, lavés 3 fois par 150 $cm^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 60 °C. On obtient ainsi 5 g de (pyridyl-3)-6 dihydro-3,4 1H-pyrrolo[2,1-c] thiazine-1,4 carbonitrile-8 sous forme de cristaux orange clair fondant à 150 °C.

L'acide N-nicotinoyl tétrahydro-2,3,5,6 thiazine-1,4 carboxylique-3 peut être préparé de la façon suivante:

Une solution de 2,8 g de N-nicotinoyl tétrahydro-2,3,5,6 thiazine-1,4 carboxylate-3 d'éthyle dans un mélange de 25 $cm^3$ d'éthanol et de 10 $cm^3$ d'une solution aqueuse de soude 2 N est agitée à une température voisine de 20 °C pendant 3 heures. Le solvant est ensuite évaporé sous pression réduite (20 mm de mercure; 2,7 kPa) à une température voisine de 50 °C. Le produit obtenu est dissous dans 50 $cm^3$ d'eau distillée et purifié par passage de cette solution sur 30 g de résine DOWEX 50 WX-2 (50–100 mesh) contenus dans une colonne de 1,6 cm de diamètre. La première fraction provenant de l'élution par de l'eau distillée, la deuxième fraction provenant de l'élution par du méthanol et la troisième fraction provenant de l'élution par de l'eau distillée sont éliminées ainsi que les deux fractions suivantes provenant de l'élution par une solution aqueuse de pyridine à 2% (v/v). Les deux fractions suivantes provenant de l'élution par une solution aqueuse de pyridine à 2% (v/v) sont réunies et concentrées à sec sous pression réduite (20 mm de mercure; 2,7 kPa) à une température voisine de 50 °C. On obtient ainsi 2,3 g de produit brut. Ce produit est dissous dans 50 $cm^3$ de méthanol bouillant et la solution obtenue est refroidie à une température voisine de 4 °C pendant 1 heure. Les cristaux apparus sont séparés par filtration, lavés 2 fois par 100 $cm^3$ au total de méthanol puis 3 fois par 45 $cm^3$ au total d'oxyde d'isopropyle et séchés sous pression réduite (20 mm de mercure; 2,7 kPa) à une température voisine de 20 °C en présence de potasse en pastilles. On obtient ainsi 1,5 g d'acide N-nicotinoyl tétrahydro-2,3,5,6 thiazine-1,4 carboxylique-3 sous forme de cristaux blancs fondant à 212 °C.

Le N-nicotinoyl tétrahydro-2,3,5,6 thiazine-1,4 carboxylate-3 d'éthyle peut être obtenu de la façon suivante:

A une solution de 8,8 g de thiazine-1,4 carboxylate-3 d'éthyle et de 10,1 g de triéthylamine dans 125 $cm^3$ de chloroforme, on ajoute, en 25 minutes à une température comprise entre 24 °C et 38 °C, 8,9 g de chlorhydrate de chlorure de nicotinoyle. On agite la solution obtenue pendant 3 heures à une température voisine de 20 °C puis ajoute successivement 10,1 g de triéthylamine puis, en 15 minutes à une température comprise entre 24 °C et 36 °C, 8,9 g de chlorhydrate de chlorure de nicotinoyle. La solution obtenue est agitée pendant 16 heures à une température voisine de 20 °C puis pendant 2 heures à l'ébullition. Le mélange réactionnel est refroidi à une température voisine de 20 °C et additionné d'un mélange de 250 $cm^3$ de chloroforme et de 100 $cm^3$ d'eau distillée. La phase organique est séparée par décantation, lavée par 100 $cm^3$ d'eau distillée puis 2 fois par 300 $cm^3$ au total d'une solution aqueuse de soude 2 N et 2 fois par 200 $cm^3$ au total d'eau distillée, séchée sur du carbonate de potassium anhydre, additionnée de 0,5 g de noir décolorant, filtrée et concentrée à sec sous pression réduite (20 mm de mercure; 2,7 kPa) à une température voisine de 60 °C. On obtient ainsi 17,5 g de produit brut. Ce produit est chromatographié sur une colonne de 6 cm de diamètre contenant 480 g de silice (0,04–0,063 mm). On élue par une mélange d'acétate d'éthyle et de méthanol (98–2 en volumes) sous une pression de 0,5 bar (51 kPa) en recueillant des fractions de 100 $cm^3$. Les 14 premières fractions sont éliminées; les 9 fractions suivantes sont réunies et concentrées à sec sous pression réduite (20 mm de mercure; 2,7 kPa) à une température voisine de 60 °C. On obtient ainsi 9,6 g de N-nicotinoyl tétrahydro-2,3,5,6 thiazine-1,4 carboxylate-3 d'éthyle sous forme d'une huile jaune.

[Rf = 0,35; chromatographie sur couche mince de gel de silice; solvant: acétate d'éthyle-méthanol (98–2 en volumes).]

Le tétrahydro-2,3,5,6 thiazine-1,4 carboxylate-3 d'éthyle peut être obtenu selon B. Belleau, J. Med. Pharm. Chem. 2, 553 (1960).

Exemple 10

On chauffe pendant 3 heures et 30 minutes à une température voisine de 95 °C une suspension de 34,5 g d'acide N-nicotinoyl tétrahydro-3,4,5,6 2H-thiazine-1,3 carboxylique-4 et 119 g de chloro-2 acrylonitrile dans 600 $cm^3$ d'anhydride acétique. La solution obtenue est refroidie sous agitation à une température voisine de 4 °C pendant 1 heure. Les cristaux apparus sont séparés par filtration, lavés 2 fois par 10 $cm^3$ au total d'anhydride acétique refroidi à une température voisine de 4 °C et 2 fois par 30 $cm^3$ au total d'oxyde d'isopropyle puis séchés sous pression réduite (20 mm de mercure; 2,7 kPa) à une température voisine de 20 °C en présence de potasse en pastilles. On obtient ainsi 16,4 g de (pyridyl-3)-6 dihydro-1,2 4H-pyrrolo [1,2-c] thiazine-1,3 carbonitrile-8 à l'état de chlorhydrate, sous forme de cristaux ocre fondant à 195 °C.

Une solution de 0,8 g de chlorhydrate de (pyridyl-3)-6 dihydro-1,2 4H-pyrrolo [1,2-c] thiazine-1,3 carbonitrile-8 dans 25 $cm^3$ d'eau distillée est amenée à un pH voisin de 10 par addition d'une solution aqueuse de soude N et extraite 3 fois par 100 $cm^3$ au total d'acétate d'éthyle. Les extraits organiques sont réunis, lavés 3 fois par 90 $cm^3$ au total d'eau distillée, séchés sur du sulfate de magnésium anhydre, additionnés de 0,1 g de noir décolorant, filtrés et concentrés à sec sous pression réduite (20 mm de mercure; 2,7 kPa) à une température voisine de 50 °C. On obtient ainsi 0,5 g de (pyridyl-3)-6 dihydro-1,2 4H-pyrrolo [1,2-c] thiazine-1,3 carbonitrile-8 sous forme de cristaux ocre fondant à 156 °C.

L'acide N-nicotinoyl tétrahydro-3,4,5,6 2H-thiazine-1,3 carboxylique-4 peut être préparé de la façon suivante:

Une solution de 37,8 g de N-nicotinoyl tétrahydro-3,4,5,6 2H-thiazine-1,3 carboxylate-4 d'éthyle dans un mélange de 80 $cm^3$ d'une solution aqueuse de soude 5 N et de 80 $cm^3$ d'éthanol est agitée à une température voisine de 20 °C pendant 16 heures. Le solvant est ensuite évaporé sous pression réduite (20 mm de mercure; 2,7 kPa) à une température voisine de 50 °C. Le résidu est dissous dans 100 $cm^3$ d'eau distillée et purifié par

passage de la solution obtenue sur 630 g de résine DOWEX 50WX-2 (50–100 mesh) contenus dans une colonne de 4,5 cm de diamètre. On élue par 4000 $cm^3$ d'eau distillée puis par 1000 $cm^3$ d'un mélange d'eau et de méthanol (50–50 en volumes) puis par 2000 $cm^3$ de méthanol puis par 2000 $cm^3$ d'eau distillée. Toutes les fractions correspondantes sont éliminées. Les 6 fractions suivantes de 1000 $cm^3$ chacune provenant de l'élution par une solution aqueuse à 2% (v/v) de pyridine sont réunies, concentrées à sec sous pression réduite (20 mm de mercure; 2,7 kPa) à une température voisine de 50 °C. Le résidu est repris par 150 $cm^3$ déthanol et le solvant est évaporé sous pression réduite (20 mm de mercure; 2,7 kPa) à une température voisine de 50 °C. Cette opération est répété 1 fois. Le résidu finalement recueilli est dissous dans 350 $cm^3$ d'un mélange bouillant d'éthanol et d'eau (60–40 en volumes). La solution obtenue est additionnée de 0,5 g de noir décolorant et filtrée à chaud; le filtrat est refroidi à une température voisine de 4 °C pendant 1 heure. Les cristaux apparus sont séparés par filtration, lavés 3 fois par 60 $cm^3$ au total d'un mélange d'éthanol et d'eau (60–40 en volumes) puis 3 fois par 60 $cm^3$ au total d'éthanol et séchés sous pression réduite (20 mm de mercure; 2,7 kPa) à une température voisine de 20 °C en présence de potasse en pastilles. On obtient ainsi 24,2 g d'acide N-nicotinoyl tétrahydro-3,4,5,6 2H-thiazine-1,3 carboxylique-4 sous forme de cristaux blancs fondant à 214 °C.

Le N-nicotinoyl tétrahydro-3,4,5,6 2H-thiazine-1,3 carboxylate-4 d'éthyle peut être préparé de la façon suivante:

A une suspension de 37,2 g de chlorhydrate de tétrahydro-3,4,5,6 2H-thiazine-1,3 carboxylate-4 d'éthyle dans 350 $cm^3$ de chloroforme, on ajoute, en 15 minutes à une température voisine de 20 °C, un mélange de 75 g de triéthylamine et de 100 $cm^3$ de chloroforme. A la solution ainsi obtenue, on ajoute, en 10 minutes à une température voisine de 20 °C, 50,4 g de chlorhydrate de chlorure de nicotinoyle. Le mélange réactionnel est chauffé à une température voisine de 65 °C pendant 1 heure et 45 minutes puis est agité à une température voisine de 20 °C pendant 16 heures. Le mélange réactionnel est lavé 3 fois par 600 $cm^3$ au total d'eau distillé puis 3 fois par 600 $cm^3$ au total d'une solution aqueuse saturée de bicarbonate de potassium, séché sur du sulfate de magnésium anhydre, additionné de 0,5 g de noir décolorant, filtré et concentré à sec sous pression réduite (20 mm de mercure; 2,7 kPa) à une température voisine de 50 °C. On obtient ainsi 52 g de produit. Ce produit est chromatographié sur une colonne de 5,2 cm de diamètre contenant 520 g de silice (0,063–0,2 mm) en recueillant des fractions de 500 $cm^3$. La première fraction provenant de l'élution par un mélange de cyclohexane et d'acétate d'éthyle (50–50 en volumes) est éliminée. Les 3 fractions suivantes provenant de l'élution par un mélange d'acétate d'éthyle et de cyclohexane (50–50 en volumes), les deux fractions suivantes provenant de l'élution par un mélange d'acétate d'éthyle et de cyclohexane (70–30 en volumes) et la fraction suivante provenant de l'élution par un mélange d'acétate d'éthyle et de cyclohexane (80–20 en volumes) sont réunies et concentrées à sec sous pression réduite (20 mm de mercure; 2,7 kPa) à une température voisine de 50 °C. On obtient ainsi 37,8 g de N-nicotinoyl tétrahydro-3,4,5,6 2H-thiazine-1,3 carboxylate-4 d'éthyle sous forme d'une huile jaune.

[Rf = 0,33; chromatographie sur couche mince de gel de silice; solvant: acétate d'éthyle-cyclohexane (80–20 en volumes).]

Le chlorhydrate de tétrahydro-3,4,5,6 2H-thiazine-1,3 carboxylate-4 d'éthyle peut être préparé de la façon suivante:

On sature pendant 4 heures à une température voisine de 20 °C une suspension de 47,7 g d'acide tétrahydro-3,4,5,6 2H-thiazine-1,3 carboxylique-4 dans 650 $cm^3$ d'éthanol par un courant d'acide chlorhydrique sec. La suspension est agitée pendant 3 jours à une température voisine de 20 °C puis est chauffée sous agitation à une température voisine de 80 °C pendant 3 heures et 20 minutes. Après refroidissement de la solution obtenue à une température voisine de 4 °C, les cristaux apparus sont séparés par filtration, lavés 3 fois par 30 $cm^3$ au total d'éthanol refroidi à une température voisine de 4 °C puis 3 fois par 120 $cm^3$ au total d'éther diéthylique et séchés sous pression réduite (20 mm de mercure; 2,7 kPa) à une température voisine de 20 °C en présence de potasse en pastilles. On obtient ainsi 37,2 g de chlorhydrate de tétrahydro-3,4,5,6 2H-thiazine-1,3 carboxylate-4 d'éthyle sous forme de cristaux blancs fondant à 185 °C.

L'acide tétrahydro-3,4,5,6 2H-thiazine-1,3 carboxylique-4 peut être préparé selon J. C. Wriston, Jr. et C. G. MacKenzie, J. Biol. Chem., 225, 607 (1957).

Exemple 11

Un mélange de 18,7 g de (pyridyl-3)-5 1H,3H-pyrrolo [1,2-c] thiazolecarbonitrile-7, 16,3 g de potasse en pastilles et 160 $cm^3$ d'éthylèneglycol est chauffée sous agitation à une température voisine de 155 °C pendant 2 heures. Après 16 heures d'agitation à une température voisine de 20 °C, le solvant est évaporé sous pression réduite (20 mm de mercure; 2,7 kPa) à une température voisine de 100 °C. Le résidu est dissous dans 100 $cm^3$ d'eau distillée et la solution obtenue est amenée à un pH voisin de 5 par addition d'une solution aqueuse d'acide chlorhydrique 2 N. Les cristaux apparus sont séparés par filtration, lavés 3 fois par 150 $cm^3$ au total d'eau distillée puis 3 fois par 150 $cm^3$ au total d'acétone et séchés sous pression réduite (20 mm de mercure; 2,7 kPa) à une température voisine de 20 °C en présence de potasse en pastilles. On obtient ainsi 17,7 g de produit brut fondant à 264 °C. Ce produit est réuni à 1,3 g de produit préparé de la même façon dans une opération antérieure et dissous dans un mélange de 650 $cm^3$ de butanol-1 et de 150 $cm^3$ de diméthylformamide préalablement chauffé à une température voisine de 115 °C. On ajoute 0,5 g de noir décolorant à la solution obtenue et on filtre à

chaud. Le filtrat est refroidi à une température voisine de 4 °C pendant 16 heures. Les cristaux apparus sont séparés par filtration, lavés 2 fois par 50 $cm^3$ au total de diméthylformamide, 3 fois par 150 $cm^3$ au total d'éthanol, 3 fois par 150 $cm^3$ au total d'oxyde d'isopropyle puis 3 fois par 150 $cm^3$ au total d'éther diéthylique et séchés sous pression réduite (20 mm de mercure; 2,7 kPa) à une température voisine de 20 °C en présence de potasse en pastilles. On obtient ainsi 16,1 g de produit fondant à 266 °C. Ce produit est mis en suspension dans 250 $cm^3$ d'eau distillée et la suspension est agitée à une température voisine de 20 °C pendant 2 heures. Les cristaux sont séparés par filtration, lavés 5 fois par 150 $cm^3$ au total d'eau distillée, 3 fois par 90 $cm^3$ au total d'éthanol, 3 fois par 90 $cm^3$ au total d'oxyde d'isopropyle puis 3 fois par 90 $cm^3$ au total d'éther diéthylique et séchés sous pression réduite (20 mm de mercure; 2,7 kPa) à une température voisine de 100 °C en présence de potasse en pastilles. On obtient ainsi 15,5 g d'acide (pyridyl-3)-5 1H,3H-pyrrolo[1,2-c] thiazolecarboxylique-7 sous forme de cristaux crème fondant à 266 °C.

Le (pyridyl-3)-5 1H,3H-pyrrolo[1,2-c] thiazolecarbonitrile-7 est préparé comme à l'exemple 3.

Exemple 12

Une suspension de 81,3 g de (pyridyl-3)-5 1H,3H-pyrrolo [1,2-c] thiazolecarbothioamide-7 de 49 g d'iodure de méthyle dans un mélange de 3110 $cm^3$ d'acétone et de 1550 $cm^3$ de diméthylformamide est agitée à une température voisine de 20 °C pendant 3 jours. Les cristaux apparus sont séparés par filtration, lavés 3 fois par 1500 $cm^3$ au total d'acétone puis 2 fois par 500 $cm^3$ au total d'éther diéthylique et séchés sous pression réduite (20 mm de mercure; 2,7 kPa) à une température voisine de 20 °C en présence de potasse en pastilles. On obtient ainsi 113 g d'iodhydrate de (pyridyl-3)-5 1H,3H-pyrrolo[1,2-c] thiazolethiocarboximidate-7 de S-méthyle sous forme de cristaux jaunes fondant à 262 °C.

Le (pyridyl-3)-5 1H,3H-pyrrolo[1,2-c] thiazolecarbothioamide-7 peut être préparé de la façon suivante:

Une suspension de 22,7 g de (pyridyl-3)-5 1H,3H-pyrrolo [1,2-c] thiazolecarbonitrile-7 dans un mélange de 14 $cm^3$ de triéthylamine et de 32 $cm^3$ de pyridine est saturée pendant 5 heures à une température voisine de 20 °C par un courant gazeux de sulfure d'hydrogène. Après agitation à une température voisine de 20 °C pendant 3 jours, on ajoute 32 $cm^3$ de pyridine au mélange réactionnel et on sature de nouveau la suspension pendant 8 heures à une température voisine de 20 °C par un courant gazeux de sulfure d'hydrogène puis on maintient l'agitation pendant 16 heures à une température voisine de 20 °C. La même opération est répétée 2 fois. On sature encore la suspension pendant 3 heures supplémentaires à une température voisine de 20 °C par un courant gazeux de sulfure d'hydrogène puis on verse le mélange réactionnel sur 500 $cm^3$ d'eau distillée. Les cristaux apparus sont séparés par filtration, lavés 4 fois par 200 $cm^3$ au total d'eau distillée puis 2 fois par 100 $cm^3$ au total d'éthanol puis 2 fois par 100 $cm^3$ au total d'oxyde d'isopropyle et séchés sous pression réduite (20 mm de mercure; 2,7 kPa) à une température voisine de 20 °C en présence de potasse en pastilles. On obtient ainsi 26 g de produit brut fondant à 230 °C. Ce produit est dissous dans 230 $cm^3$ de diméthylformamide à une température voisine de 100 °C. La solution obtenue est additionnée de 1 g de noir décolorant et filtrée à chaud; le filtrat est refroidi à une température voisine de 4 °C pendant 2 heures. Les cristaux apparus sont séparés par filtration, lavés 2 fois par 40 $cm^3$ au total de diméthylformamide, 3 fois par 150 $cm^3$ au total d'éthanol puis 3 fois par 150 $cm^3$ au total d'oxyde d'isopropyle et séchés sous pression réduite (20 mm de mercure; 2,7 kPa) à une température voisine de 20 °C en présence de potasse en pastilles. On obtient ainsi 21 g de (pyridyl-3)-5 1H,3H-pyrrolo[1,2-c] thiazolecarbothioamide-7 sous forme de cristaux jaunes fondant à 243 °C.

Le (pyridyl-3)-5 1H,3H-pyrrolo[1,2-c] thiazolecarbonitrile-7 est préparé comme décrit à l'exemple 3.

Exemple d'application 1

A un mélange de 10,2 g d'acide (pyridyl-3)-3 1H,3H-pyrrolo[1,2-c] thiazolecarboxylique-7 et de 10,1 g de N,N'-carbonyldiimidazole dans 150 $cm^3$ de tétrahydrofuranne anhydre agité sous azote sec à une température voisine de 20 °C pendant 1 heure et 20 minutes, on ajoute une solution de 9,5 g de diéthylamino-2 éthylamine dans 50 $cm^3$ de tétrahydrofuranne anhydre en 10 minutes à une température voisine de 25 °C. Après une heure d'agitation à une température voisine de 20 °C, on évapore le solvant sous pression réduite (20 mm de mercure; 2,7 kPa) à une température voisine de 40 °C. Le résidu obtenu est repris par 700 $cm^3$ d'eau distillée et le mélange est extrait 5 fois par 750 $cm^3$ au total d'acétate d'éthyle. Les extraits organiques sont réunis, lavés 5 fois avec 500 $cm^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 40 °C. On obtient ainsi 12,4 g de produit brut. Ce produit, réuni avec 2 g de produit préparé de la même manière dans une autre opération antérieure, est dissous dans 100 $cm^3$ d'un mélange bouillant de cyclohexane et d'acétate d'éthyle (50–50 en volumes). La solution obtenue est additionnée de 0,5 g de noir décolorant puis filtrée à chaud; le filtrat est refroidi à une température voisine de 4 °C pendant 2 heures. Les cristaux apparus sont séparés par filtration, lavés 3 fois par 45 $cm^3$ au total d'oxyde d'isopropyle et séchés sour pression réduite (20 mm de mercure; 2,7 kPa) à une température voisine de 20 °C en présence de potasse en pastilles. On obtient ainsi 9,8 g de N-(diéthylamino-2 éthyl) (pyridyl-3)-3 1H,3H-pyrrolo[1,2-c] thiazolecarboxamide-7 sous forme de cristaux blancs fondant à 129 °C.

L'acide (pyridyl-3)-3 1H,3H-pyrrolo[1,2-c] thiazolecarboxylique-7 peut être obtenu comme à l'exemple 1.

Exemple d'application 2

Une solution de 2 g d'acide (pyridyl-3)-3 1H,3H-pyrrolo [1,2-c] thiazolecarboxylique-7 et de 1,45 g de N,N'-carbonyldiimidazole dans 40 $cm^3$ de tétrahydrofuranne anhydre est agitée à une température voisine de 20 °C pendant 4 heures. La solution obtenue est additionnée de 0,5 g de noir décolorant, filtrée et concentrée à sec sous pression réduite (20 mm de mercure; 2,7 kPa) à une température voisine de 45 °C. Le résidu est repris par 100 $cm^3$ d'eau distillée et la suspension obtenue est agitée à une température voisine de 20 °C pendant 30 minutes. Les cristaux sont séparés par filtration, lavés 3 fois par 60 $cm^3$ au total d'eau distillée et séchés sous pression réduite (20 mm de mercure; 2,7 kPa) à une température voisine de 20 °C en présence de potasse en pastilles. On obtient ainsi 2 g de produit brut. Ce produit est dissous dans 35 $cm^3$ d'isopropanol bouillant. La solution obtenue est refroidie à une température voisine de 4 °C pendant 1 heure. Les cristaux apparus sont séparés par filtration, lavés 2 fois par 20 $cm^3$ au total d'isopropanol refroidi à une température voisine de 4 °C et 2 fois par 20 $cm^3$ au total d'oxyde d'isopropyle puis séchés sous pression réduite (20 mm de mercure; 2,7 kPa) à une température voisine de 20 °C en présence de potasse en pastilles. On obtient ainsi 1,7 g d'(imidazolyl-1 carbonyl)-7(pyridyl-3)-3 1H,3H-pyrrolo [1,2-c] thiazole sous forme de cristaux crème fondant à 117 °C.

L'acide (pyridyl-3)-3 1H,3H-pyrrolo [1,2-c] thiazolecarboxylique-7 est préparé comme à l'exemple 1.

Exemple d'application 3

On chauffe pendant 5 heures et 30 minutes à une température voisine de 150 °C une solution de 16 g d'(imidazolyl-1 carbonyl)-7 (pyridyl-3)-3 1H,3H pyrrolo [1,2-c] thiazole et de 10,2 g d'amino-2 pyridine dans 150 $cm^3$ de diméthylformamide anhydre. La solution est alors concentrée à sec sous pression réduite (20 mm de mercure; 2,7 kPa) à une température voisine de 80 °C et l'huile résiduelle est reprise par 500 $cm^3$ d'eau distillée. Des cristaux apparaissent. La suspension est agitée à une température voisine de 20 °C pendant 16 heures. Les cristaux sont séparés par filtration, lavés 3 fois par 300 $cm^3$ au total d'eau distillée et séchés sous pression réduite (20 mm de mercure; 2,7 kPa) à une température voisine de 20 °C en présence de potasse en pastilles. On obtient ainsi 14,6 g de produit brut fondant à 141 °C. Ce produit est dissous dans 75 $cm^3$ d'éthanol bouillant. La solution obtenue est additionnée de 0,5 g de noir décolorant et filtrée à chaud. Le filtrat est refroidi à une température voisine de 4 °C pendant 1 heure. Les cristaux apparus sont séparés par filtration, lavés 2 fois par 10 $cm^3$ au total d'éthanol refroidi à une température voisine de 4 °C et 3 fois par 15 $cm^3$ au total d'éther diéthylique puis séchés sous pression réduite (20 mm de mercure; 2,7 kPa) à une température voisine de 20 °C en présence de potasse en pastilles. On obtient ainsi 11,6 g de N-(pyridyl-2) (pyridyl-3)-3 1H,3H-pyrrolo [1,2-c] thiazolecarboxamide-7 sous forme de cristaux beiges fondant à 145 °C.

L'(imidazolyl-1 carbonyl)-7 (pyridyl-3)-3 1H,3H-pyrrolo [1,2-c] thiazole est préparé comme à l'exemple d'application 2.

Exemple d'application 4

A une solution d'éthoxymagnésien du malonate de diéthyle dans 65 $cm^3$ d'un mélange d'éther et d'éthanol (3–1 en volumes), préparée à partir de 1,34 g de magnésium et de 8,8 g de malonate de diéthyle, on ajoute 5,1 g de triéthylamine; à la suspension obtenue, on ajoute en 15 minutes à une température comprise entre 25 °C et 30 °C 15 g de chlorhydrate de chloroformyl-7 (pyridyl-3)-3 1H,3H-pyrrolo [1,2-c] thiazole, puis on dilue par 35 $cm^3$ de tétrahydrofuranne anhydre et agite à une température voisine de 20 °C pendant 16 heures. Le mélange réactionnel est alors repris par 25 $cm^3$ d'une solution aqueuse d'acide chlorhydrique 2 N extrait 5 fois par 750 $cm^3$ au total d'acétate d'éthyle. Les extraits organiques sont réunis, lavés 5 fois par 250 $cm^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 50 °C. On obtient ainsi 19 g de produit fondant à 110 °C. Ce produit est dissous dans un mélange de 25 $cm^3$ d'acide acétique, de 15 $cm^3$ d'eau distillée et de 3 $cm^3$ d'acide sulfurique concentré. La solution obtenue est chauffée à une température voisine de 100 °C pendant 9 heures et 30 minutes puis est refroidie à une température voisine de 20 °C, diluée par 150 $cm^3$ d'eau distillée, additionnée de 0,5 g de noir décolorant, filtrée, amenée à un pH voisin de 9 par addition de carbonate de sodium et extraite 3 fois par 450 $cm^3$ au total d'acétate d'éthyle. Les extraits organiques sont réunis, lavés 3 fois par 450 $cm^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 45 °C. On obtient ainsi 9,5 g de produit. Ce produit est chromatographé sur une colonne de 6 cm de diamètre contenant 480 g de silice (0,04–0,063 mm) en éluant par des mélanges de cyclohexane et d'acétate d'éthyle sous une pression de 0,5 bar (51 kPa) et en recueillant des fractions de 200 $cm^3$. Les 9 premières fractions provenant de l'élution par une mélange d'acétate d'éthyle et de cyclohexane (80–20 en volumes) sont éliminées. Les 9 fractions suivantes provenant de l'élution par un mélange d'acétate d'éthyle et de cyclohexane (85–15 en volumes) sont réunies et concentrées à sec sous pression réduite (20 mm de mercure; 2,7 kPa) à une température voisine de 50 °C. On obtient ainsi 7,2 g de produit. Ce produit est dissous dans 30 $cm^3$ d'isopropanol bouillant. La solution obtenue est additionnée de 0,5 g de noir décolorant et filtrée à chaud. Le filtrat est

refroidi à une température voisine de 4 °C pendant 1 heure. Les cristaux apparus sont séparés par filtration, lavés 3 fois par 15 cm$^3$ au total d'isopropanol refroidi à une température voisine de 4 °C et séchés sous pression réduite (20 mm de mercure; 2,7 kPa) à une température voisine de 20 °C en présence de potasse en pastilles. On obtient ainsi 5,7 g d'acétyl-7 (pyridiyl-3)-3 1H,3H-pyrrolo [1,2-c] thiazole sous forme de cristaux crème fondant à 100 °C.

Le chlorhydrate de chlorofromyl-7 (pyridyl-3)-3 1H,3H-pyrrolo [1,2-c] thiazole est préparé de la manière suivante:

Une suspension de 9,8 g d'acide (pyridyl-3)-3 1H,3H-pyrrolo [1,2-c] thiazolecarboxylique-7 dans un mélange de 23,8 g de chlorure de thionyle, de 0,1 cm$^3$ de diméthylformamide et de 100 cm$^3$ de dichloro-1,2 éthane est chauffée à l'ébullition pendant 1 heure et 15 minutes. Après refroidissement du mélange réactionnel, le solvant est évaporé sous pression réduite (20 mm de mercure; 2,7 kPa) à une température voisine de 50 °C. Le résidu obtenu est mis en suspension dans 100 cm$^3$ de cyclohexane anhydre et les cristaux sont séparés par filtration, lavés 2 fois par 10 cm$^3$ au total de cyclohexane anhydre et séchés sous pression réduite (20 mm de mercure; 2,7 kPa) à une température voisine de 20 °C. On obtient ainsi 12,1 g de chlorhydrate de chloroformyl-7 (pyridyl-3)-3 1H,3H-pyrrolo [1,2-c] thiazole sous forme de cristaux ocre fondant à 185 °C.

L'acide (pyridyl-3)-3 1H,3H-pyrrolo [1,2-c] thiazolecarboxylique-7 est préparé comme à l'exemple 1.

L'éthoxymagnésien du malonate de diéthyle est préparé selon G. A. Reynolds et C. R. Hauser, Org. Synth. Coll. Vol. 4, 708 (1963).

Exemple d'application 5

Une suspension de 9,5 g d'iodhydrate de (pyridyl-3)-3 1H, 3H-pyrrolo [1,2-c] thiazolethiocarboxyimidate-7 de S-méthyle et de 1,6 g de N,N-diméthylhydrazine dans 125 cm$^3$ d'éthanol est chauffée à une température voisine de 78 °C pendant 4 heures et 30 minutes. La solution obtenue est concentrée à sec sous pression réduite (20 mm de mercure; 2,7 kPa) à une température voisine de 60 °C. Le résidu obtenu est dissous dans 250 cm$^3$ d'eau distillée et la solution est extraite 3 fois par 300 cm$^3$ au total d'acétate d'éthyle, additionnée de 0,5 g de noir décolorant, filtrée, amenée à un pH voisin de 10 par addition d'une solution aqueuse de soude 10 N et extraite 3 fois par 300 cm$^3$ au total d'acétate d'éthyle. Les extraits organiques sont réunis, lavés 3 fois par 300 cm$^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 50 °C. On obtient ainsi 4,8 g de produit brut que l'on chromatographie sur une colonne de 4 cm de diamètre contenant 320 g de silice (0,04–0,063 mm). On élue sous une pression de 0,5 bar (51 kPa) par des mélanges de chlorure de méthylène et de méthanol en recueillant des fractions de 100 cm$^3$. Les 13 premières fractions provenant de l'élution par un mélange de chlorure de méthylène et de méthanol (90–10 en volumes) sont éliminées. Les 4 fractions suivantes provenant de l'élution par un mélange de chlorure de méthylène et de méthanol (90–10 en volumes), les 9 fractions suivantes provenant de l'élution par un mélange de chlorure de méthylène et de méthanol (80–20 en volumes) et les 10 fractions suivantes provenant de l'élution par un mélange de chlorure de méthylène et de méthanol (50–50 en volumes) sont réunies et concentrées à sec sous pression réduite (20 mm de mercure; 2,7 kPa) à une température voisine de 60 °C. On obtient ainsi 3,1 g de produit que l'on dissout dans 20 cm$^3$ d'éthanol. La solution trouble obtenue est filtrée et additionnée de 8,1 cm$^3$ d'une solution éthanolique de gaz chlorhydrique 5,35 N; le chlorhydrate obtenu est précipté de sa solution par addition de 2 cm$^3$ d'éther diéthylique. Après refroidissement à une température voisine de 4 °C pendant 16 heures, les cristaux sont séparés par filtration, lavés 5 fois par 75 cm$^3$ au total d'un mélange d'éther diéthylique et d'éthanol (50–50 en volumes) et 5 fois par 75 cm$^3$ au total d'éther diéthylique puis séchés sous pression réduite (20 mm de mercure; 2,7 kPa) à une température voisine de 20 °C en présence de potasse en pastilles. On obtient ainsi 2,7 g de dichlorhydrate de (pyridyl-3)-3 1H,3H-pyrrolo [1,2-c] thiazolecarboxamide-7-diméthylhydrazone sous forme de cristaux crème fondant à 195 °C.

L'iodhydrate de (pyridyl-3)-3 1H,3H-pyrrolo [1,2-c] thiazolecarboximidate-7 de S-méthyle peut être préparé comme à l'exemple 2.

Exemple d'application 6

Une suspension de 11,35 g de (pyridyl-3)-5 1H,3H-pyrrolo[1,2-c] thiazolecarbonitrile-7 et de 14 g de potasse en poudre dans 100 cm$^3$ d'alcool tert.butylique est chauffée à 85 °C pendant 1 heure. Après 16 heures d'agitation à une température voisine de 20 °C, le mélange réactionnel est versé sur 2 litres d'eau distillée. La suspension est agitée à une température voisine de 20 °C pendant 15 minutes puis les cristaux apparus sont séparés par filtration, lavés 8 fois par 1200 cm$^3$ au total d'eau distillée puis 3 fois par 150 cm$^3$ au total d'éthanol et séchés sous pression réduite (20 mm de mercure; 2,7 kPa) à une température voisine de 20 °C en présence de potasse en pastilles. On obtient ainsi 10,5 g de produit brut qui sont réunis à 3,9 g de produit préparé de la même façon dans une opération antérieure et dissous dans 850 cm$^3$ d'éthanol bouillant. La solution est additionnée de 0,5 g de noir décolorant et filtrée à chaud. Le filtrat est refroidi à une température voisine de 4 °C pendant 3 jours. Les cristaux apparus sont séparés par filtration, lavés 3 fois par 30 cm$^3$ au total d'éthanol refroidi à une température voisine de 4 °C et séchés sous pression réduite (20 mm de mercure; 2,7 kPa) à une température voisine de 20 °C en présence de potasse en pastilles. On obtient ainsi 11,3 g de (pyridyl-3)-5 1H,3H-pyrrolo[1,2-c] thiazolecarboxamide-7 sous forme de cristaux crème fondant à 215 °C.

Le (pyridyl-3)-5 1H,3H-pyrrolo[1,2-c] thiazolecarbonitrile-7 est préparé comme décrit à l'exemple 3.

Exemple d'application 7

Une suspension de 22,7 g de (pyridyl-3)-5 1H,3H-pyrrolo [1,2-c] thiazolecarbonitrile-7 dans un mélange de 14 $cm^3$ de triéthylamine et de 32 $cm^3$ de pyridine est saturée pendant 5 heures à une température voisine de 20 °C par un courant gazeux de sulfure d'hydrogène. Après agitation à une température voisine de 20 °C pendant 3 jours, on ajoute 32 $cm^3$ de pyridine au mélange réactionnel et on sature de nouveau la suspension pendant 8 heures à une température voisine de 20 °C par un courant gazeux de sulfure d'hydrogène puis on maintient l'agitation pendant 16 heures à une température voisine de 20 °C. La même opération est répétée 2 fois. On sature encore la suspension pendant 3 heures supplémentaires à une température voisine de 20 °C par un courant gazeux de sulfure d'hydrogène puis on verse le mélange réactionnel sur 500 $cm^3$ d'eau distillée. Les cristaux apparus sont séparés par filtration, lavés 4 fois par 200 $cm^3$ au total d'eau distillée puis 2 fois par 100 $cm^3$ au total d'éthanol puis 2 fois par 100 $cm^3$ au total d'oxyde d'isopropyle et séchés sous pression réduite (20 mm de mercure; 2,7 kPa) à une température voisine de 20 °C en présence de potasse en pastilles. On obtient ainsi 26 g de produit brut fondant à 230 °C. Ce produit est dissous dans 250 $cm^3$ de diméthylformamide à une température voisine de 100 °C. La solution obtenue est additionnée de 1 g de noir décolorant et filtrée à chaud; le filtrat est refroidi à une température voisine de 4 °C pendant 2 heures. Les cristaux apparus sont séparés par filtration, lavés 2 fois par 40 $cm^3$ au total de diméthylformamide, 3 fois par 150 $cm^3$ au total d'éthanol puis 3 fois par 150 $cm^3$ au total d'oxyde d'isopropyle et séchés sous pression réduite (20 mm de mercure; 2,7 kPa) à une température voisine de 20 °C en présence de potasse en pastilles. On obtient ainsi 21 g de (pyridyl-3)-5 1H,3H-pyrrolo[1,2-c] thiazolecarbothioamide-7 sous forme de cristaux jaunes fondant à 243 °C.

Le (pyridyl-3)-5 1H,3H-pyrrolo[1,2-c] thiazolecarbonitrile-7 est préparé comme décrit à l'exemple 3.

Exemple d'application 8

Une suspension de 14,6 g de (pyridyl-3)-5 dihydro-2,3 1H-pyrrolizinecarbonitrile-7 et de 23,1 g de potasse en poudre dans 140 $cm^3$ d'alcool tert-butylique est chauffée à 82 °C pendant 2 heures. Durant cette période, on observe un passage par une phase homogène limpide au bout de 10 minutes, suivi d'une précipitation 20 minutes plus tard. Aprés refroidissement à une température voisine de 20 °C, le solvant est évaporé sous pression réduite (20 mm de mercure; 2,7 kPa) à une température voisine de 60 °C. Le résidu cristallisé obtenu est mis en suspension dans 250 $cm^3$ d'eau distillée et agité à une température voisine de 20 °C pendant 30 minutes. Les cristaux sont séparés par filtration, lavés 4 fois par 200 $cm^3$ au total d'eau distillée puis 3 fois par 150 $cm^3$ au total d'oxyde d'isopropyle et séchés sous pression réduite (20 mm de mercure; 2,7 kPa) à une température voisine de 20 °C en présence de potasse en pastilles. On obtient ainsi 15,5 g de produit brut fondant à 206 °C. Ce produit est dissous dans 150 $cm^3$ d'éthanol bouillant. La solution obtenue est additionnée de 0,5 g de noir décolorant et filtrée; le filtrat est refroidi à une température voisine de 10 °C pendant 2 heures. Les cristaux sont séparés par filtration et séchés sous pression réduite (20 mm de mercure; 2,7 kPa) à une température voisine de 20 °C en présence de potasse en pastilles. On obtient ainsi 9,6 g de (pyridyl-3)-5 dihydro-2,3 1H-pyrrolizinecarboxamide-7 sous forme de cristaux crème fondant à 210 °C.

Le (pyridyl-3)-5 dihydro-2,3 1H-pyrrolizinecarbonitrile-7 peut être préparé comme à l'exemple 4.

Exemple d'application 9

A une suspension de 12 g de chlorhydrate de chloroformyl-7 (pyridyl-3)-5 1H,3H-pyrrolo[1,2-c] thiazole dans 200 $cm^3$ de chlorure de méthylène, on ajoute, en 20 minutes à une température voisine de 20 °C, une solution de 13,9 g de diéthylamino-2 éthylamine dans 50 $cm^3$ de chlorure de méthylène. La solution obtenue est agitée pendant 16 heures à une température voisine de 20 °C. Un produit précipite. On ajoute alors 250 $cm^3$ de chlorure de méthylène et 100 $cm^3$ d'une solution aqueuse de soude 2 N; la phase organique est séparée par décantation, lavée par 100 $cm^3$ d'une solution aqueuse de soude 2 N puis 3 fois par 600 $cm^3$ au total d'eau distillée, séchée sur du carbonate de potassium anhydre, additionnée de 0,5 g de noir décolorant, filtrée et concentrée à sec sous pression réduite (20 mm de mercure; 2,7 kPa) à une température voisine de 60 °C. On obtient ainsi 12 g de produit brut. Ce produit est dissous dans 60 $cm^3$ d'acétonitrile bouillant. La solution obtenue est additionnée de 0,5 g de noir décolorant et filtrée à chaud; le filtrat est refoidi à une température voisine de 4 °C pendant 2 heures. Les cristaux apparus sont séparés par filtration, lavés 2 fois par 20 $cm^3$ au total d'acétonitrile refroidi à une température voisine de 4 °C puis 3 fois par 150 $cm^3$ au total d'oxyde d'isopropyle et séchés sous pression réduite (20 mm de mercure; 2,7 kPa) à une température voisine de 20 °C en présence de potasse en pastilles. On obtient ainsi 6,4 g de N-(diéthylamino-2 éthyl) (pyridyl-3)-5 1H,3H-pyrrolo[1,2-c] thiazolecarboxamide-7 sous forme de cristaux beige clair fondant à 106 °C.

Le chlorhydrate de chloroformyl-7 (pyridyl-3)-5 1H,3H-pyrrolo[1,2-c] thiazole est préparé comme de la façon suivante:

Une suspension de 8,8 g d'acide (pyridyl-3)-5 1H,3H-pyrrolo[1,2-c] thiazolecarboxylique-7 dans un mélange de 6,25 $cm^3$ de chlorure de thionyle, 0,05 $cm^3$ de diméthylformamide et de 100 $cm^3$ de dichloro-1,2 éthane est chauffée au reflux sous agitation pendant 2 heures et 30 minutes. Le mélange réactionnel est refroidi à une température voisine de 20 °C et concentré à sec sous pression réduite (20 mm de mercure; 2,7 kPa) à une température voisine de 60 °C. Le résidu obtenu est mis en suspension dans 150 $cm^3$ de cyclohexane et le solvant est évaporé sous pression réduite (20 mm de mercure; 2,7 kPa) à une température voisine de 60 °C. La même opération est répétée 2 fois. On obtient ainsi 10 g de chlorhydrate de chloroformyl-7 (pyridyl-3)-5 pyrrolo[1,2-c] thiazole sous forme de cristaux crème fondant à 220 °C.

L'acide (pyridyl-3)-5 1H,3H-pyrrolo [1,2-c] thiazolecarboxylique-7 peut être préparé comme décrit à l'exemple 11.

Exemple d'application 10

Une suspension de 20,2 g d'iodhydrate de (pyridyl-3)-5 1H,3H-pyrrolo[1,2-c] thiazolethiocarboximidate-7 de S-méthlye et de 3,4 g de N,N-diméthylhydrazine dans 100 $cm^3$ d'éthanol est chauffée à l'ébullition pendant 5 heures et 30 minutes puis filtrée à chaud. Après refroidissement à une température voisine de 20 °C, on ajoute 350 $cm^3$ d'éther diéthylique au filtrat. La suspension obtenue est agitée à une température voisine de 20 °C pendant 30 minutes. Les cristaux apparus sont séparés par filtration, lavés 2 fois par 100 $cm^3$ au total d'un mélange d'éthanol et d'éther diéthylique (50–50 en volumes) et séchés sous pression réduite (20 mm de mercure; 2,7 kPa) à une température voisine de 20 °C en présence de potasse en pastilles. Le produit obtenu est mis en suspension dans un mélange de 300 $cm^3$ d'eau et de 300 $cm^3$ d'acétate d'éthyle. On ajoute 100 $cm^3$ d'une solution aqueuse de soude 10 N à cette suspension. La phase organique est séparée par décantation et la phase aqueuse est extraite 2 fois par 300 $cm^3$ au total d'acétate d'éthyle. Les extraits organiques sont réunis, lavés 3 fois par 300 $cm^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 50 °C. On obtient ainsi 9,3 g de produit brut fondant à 168 °C. Ce produit est dissous dans 100 $cm^3$ d'éthanol bouillant. La solution obtenue est additionnée de 0,5 g de noir décolorant et filtrée à chaud; le filtrat est refroidi à une température voisine de 4 °C pendant 1 heure. Les cristaux apparus sont séparés par filtration, lavés 3 fois par 30 $cm^3$ au total d'éthanol refroidi à une température voisine de 4 °C et 3 fois par 60 $cm^3$ au total d'oxyde d'isopropyle et séchés sous pression réduite (20 mm de mercure; 2,7 kPa) à une température voisine de 20 °C en présence de potasse en pastilles. On obtient ainsi 4,95 g de (pyridyl-3)-5 1H,3H-pyrrolo[1,2-c] thiazolecarboxamide-7 diméthylhydrazone sous forme de cristaux crème fondant à 170 °C.

L'iodhydrate de (pyridyl-3)-5 1H,3H-pyrrolo[1,2-c] thiazolecarboximidate-7 de S-méthyle est préparé comme à l'exemple 12.

Exemple d'application 11

On agite à 30 °C pendant 3 jours une suspension de 20,2 g d'iodhydrate de (pyridyl-3)-5 1H,3H-pyrrolo[1,2-c] thiazolethiocarboximidate-7 de S-méthyle, de 8,6 g de pipéridine et de 10,5 g d'acide acétique dans 500 $cm^3$ de chloroforme. On ajoute alors à la suspension 360 $cm^3$ d'une solution aqueuse de soude 2,8 N et 200 $cm^3$ de chloroforme. La phase organique est séparée par décantation et la phase aqueuse est extraite 2 fois par 1000 $cm^3$ au total de chloroforme. Les extraits organiques sont réunis, lavés 3 fois par 750 $cm^3$ au total d'eau distillée, séchés sur du sulfate de magnésium anhydre, additionnés de 0,5 g de noir décolorant et concentrés à sec sous pression réduite (20 mm de mercure; 2,7 kPa) à une température voisine de 60 °C. On obtient 15 g de produit qui est repris par 200 °C d'acétate d'éthyle bouillant. Après refroidissement à une température voisine de 20 °C, les cristaux apparus sont séparés par filtration et le filtrat est concentré à sec sous pression réduite (20 mm de mercure; 2,7 kPa) à une température de 60 °C. On obtient ainsi 14,1 g de produit. Ce produit est dissous dans 130 $cm^3$ d'éthanol et la solution obtenue est additionnée de 19,3 $cm^3$ d'une solution éthanolique d'acide chlorhydrique 4,7 N puis est refroidie à une température voisine de 4 °C pendant 1 heure. Les cristaux apparus sont séparés par filtration, lavés 3 fois par 75 $cm^3$ au total d'éthanol et 3 fois par 150 $cm^3$ au total d'éther diéthylique et séchés sous pression réduite (20 mm de mercure; 2,7 kPa) à une température voisine de 20 °C en présence de potasse en pastilles. On obtient ainsi 13,7 g de produit brut à l'état de dichlorhydrate fondant à 264 °C. Ce produit est réuni à 2,4 g de produit préparé dans une opération antérieure et est dissous dans 200 $cm^3$ d'eau distillée. On ajoute à la solution obtenue 84 $cm^3$ d'une solution aqueuse de soude 1 N et 250 $cm^3$ d'acétate d'éthyle. La phase organique est séparée par décantation et la phase aqueuse est extraite 2 fois par 200 $cm^3$ au total d'acétate d'éthyle. Les extraits organiques sont réunis, séchés sur du carbonate de potassium anhydre, additionnés de 0,5 g de noir décolorant, filtrés et concentrés à sec sous pression réduite (20 mm de mercure; 2,7 kPa) à une température voisine de 60 °C. On obtient ainsi 13 g de produit. Ce produit est chromatographié sur une colonne de 2,4 cm de diamètre contenant 65 g de silice (0,063–0,2 mm) en éluant par des mélanges d'acétonitrile et d'ammoniaque (d = 0,92) et en recueillant des fractions de 100 $cm^3$. Les 2 premières fractions provenant de l'élution par un mélange d'acétonitrile et d'ammoniaque (95–5 en volumes) sont éliminées. La troisième fraction provenant de l'élution par un mélange d'acétonitrile et d'ammoniaque (95–5 en volumes) et les 9 fractions suivantes provenant de l'élution par un mélange d'acétonitrile et d'ammoniaque (90–10 en volumes) sont réunies et concentrés à sec sous pression réduite (20 mm de mercure; 2,7 kPa) à une température voisine de 40 °C. On obtient 11,3 g de produit. Ce produit est chromatographié à nouveau sur une colonne de 6 cm de diamètre contenant 480 g de silice (0,04–0,063 mm). On élue par un mélange de chlorure de méthylène, de méthanol et d'ammoniaque à 20% (12–6–1 en volumes) sous une pression de 0,5 bar (51 kPa) en recueillant des fractions de 100 $cm^3$. Les 7 pre-

mières fractions sont éliminées, les 14 fractions suivantes sont réunies et concentrées à sec sous pression réduite (20 mm de mercure; 2,7 kPa) à une température voisine de 40 °C. On obtient ainsi 9,6 g de produit. Ce produit est repris dans 350 $cm^3$ d'acétate d'éthyle bouillant et la suspension obtenue est filtrée à chaud; le filtrat est ensuite refroidi à une température voisine de 20 °C et concentré à sec sous pression réduite (20 mm de mercure; 2,7 kPa) à une température voisine de 60 °C. On obtient ainsi 9,1 g de produit. Ce produit est dissous dans 100 $cm^3$ d'éthanol. La solution obtenue est additionnée de 12,4 $cm^3$ d'une solution éthanolique d'acide chlorhydrique 4,7 N et est refroidie à une température voisine de 4 °C pendant 2 heures. Les cristaux apparus sont séparés par filtration, lavés 2 fois par 50 $cm^3$ du total d'éthanol refroidi à une température voisine de 4 °C puis 3 fois par 150 $cm^3$ au total d'éther diéthylique et séchés sous pression réduite (20 mm de mercure; 2,7 kPa) à une température voisine de 20 °C en présence de potasse en pastilles. On obtient ainsi 9,7 g de produit. Ce produit est dissous dans un mélange bouillant de 175 $cm^3$ d'éthanol et de 10 $cm^3$ d'eau distillée. La solution obtenue est additionnée de 0,5 g de noir décolorant et filtrée à chaud; le filtrat est refroidi à une température voisine de 4 °C pendant 3 heures. Les cristaux apparus sont séparés par filtration, lavés 3 fois par 30 $cm^3$ au total d'éthanol et 3 fois par 75 $cm^3$ au total d'éther diéthylique et séchés sous pression réduite (20 mm de mercure; 2,7 kPa) à une température voisine de 20 °C en présence de potasse en pastilles. On obtient ainsi 4,5 g de dichlorhydrate de pipéridinocarbonimidoyl-7 (pyridyl-3)-5 1H,3H-pyrrolo[1,2-c] thiazole sous forme de cristaux jaune pâle fondant à 284 °C.

L'iodhydrate de (pyridyl-3)-5 1H,3H-pyrrolo[1,2-c] thiazolethiocarboximidate-7 de S-méthyle est préparé comme à l'exemple 12.

Exemple d'application 12

Une suspension de 4,9 g de (pyridyl-3)-6 dihydro-3,4 1H-pyrrolo[1,2-c] thiazine-1,4 carbonitrile-8 et de 6,7 g de potasse en poudre dans 50 $cm^3$ d'alcool tert-butylique est chauffée à l'ébullition pendant 1 heure et 15 minutes. Le solvant est ensuite évaporé sous pression réduite (20 mm de mercure; 2,7 kPa) à une température voisine de 60 °C puis le résidu est mis en suspension dans 150 $cm^3$ d'eau distillé. Les cristaux apparus sont séparés par filtration, lavés 3 fois par 150 $cm^3$ au total d'eau distillée et séchés sous pression réduite (20 mm de mercure; 2,7 kPa) à une température voisine de 20 °C. On obtient 5,2 g de produit brut fondant à 186 °C. Ce produit est réuni avec 4,5 g de produit préparé de la même façon dans des opérations antérieures et est dissous dans 250 $cm^3$ d'éthanol bouillant. La solution obtenue est additionnée de 0,5 g de noir décolorant puis filtrée à chaud; le filtrat est refroidi à une température voisine de 4 °C pendant 2 heures. Les cristaux apparus sont séparés par filtration, lavés 2 fois par 50 $cm^3$ au total d'éthanol refroidi à une température voisine de 4 °C puis 3 fois par 75 $cm^3$ au total d'éther diéthylique et séchés sous pression réduite (20 mm de mercure; 2,7 kPa) à une température voisine de 20 °C en présence de potasse en pastilles. On obtient ainsi 7,1 g de (pyridyl-3)-6 dihydro-3,4 1H-pyrrolo[2,1-c] thiazine-1,4 carboxamide-8 sous forme de cristaux jaunes fondant à 192 °C.

Le (pyridyl-3)-6 dihydro-3,4 1H-pyrrolo[2,1-c] thiazine-1,4 carbonitrile-8 peut être préparé comme à l'exemple 9.

Exemple d'application 13

En opérant comme à l'exemple d'application 12 mais à partir de (pyridyl-3)-6 dihydro-1,2 4H-pyrrolo [1,2-c] thiazine-1,3 carbonitrile-8 (préparé comme décrit à l'exemple 10) on peut obtenir la (pyridyl-3)-6 dihydro-1,2 4H-pyrrolo [1,2-c] thiazine-1,3 carboxamide sous forme de cristaux blancs fondant à 220 °C.

Exemple d'application 14

Une suspension de 11,2 g de potasse en poudre et de 10,2 g d'un mélange (dans la proportion 69–31) de cyano-6 et cyano-7 [(pyridyl-3)-2 vinyl]-5 1H,3H-pyrrolo[1,2-c] thiazole dans 110 $cm^3$ d'alcool tert-butylique est chauffée au reflux pendant une heure. Le solvant est ensuite évaporé sous pression réduite (20 mm de mercure; 2,7 kPa) à une température voisine de 50 °C. Après addition de 200 $cm^3$ d'eau distillée, les cristaux apparus sont séparés par filtration, lavés quatre fois par 120 $cm^3$ au total d'eau distillée et séchés sous pression réduite (20 mm de mercure; 2,7 kPa) à une température voisine de 20 °C en présence de potasse en pastilles. On obtient ainsi 11 g de produit brut. Ce produit est chromatographié sur une colonne de 3,2 cm de diamètre contenant 110 g de silice (0,063–0,2 mm) en recueillant des fractions de 1000 $cm^3$. La première fraction provenant de l'élution par du chlorure de méthylène pur, la deuxième fraction provenant de l'élution par un mélange de chlorure de méthylène et de méthanol (97,5–2,5 en volumes), la troisième fraction provenant de l'élution par un mélange de chlorure de méthylène et de méthanol (95–5 en volumes) et la quatrième fraction provenant de l'élution par un mélange de chlorure de méthylène et de méthanol (92,5–7,5 en volumes) sont éliminées. La cinquième fraction provenant de l'élution par un mélange de chlorure de méthylène et de méthanol (90–10 en volumes) et la sixième fraction provenant de l'élution par un mélange de chlorure de méthylène et de méthanol (85–15 en volumes) sont réunies et concentrées à sec sous pression réduite (20 mm de mercure; 2,7 kPa) à une température voisine de 40 °C. On obtient ainsi 3,7 g de produit fondant à 180 °C. Ce produit est dissous dans 700 $cm^3$ d'acétonitrile bouillant. Après refroidissement à une température voisine de 4 °C pendant une heure, les cristaux apparus sont séparés par filtration et séchés sous pression réduite (20 mm de mercure; 2,7 kPa) à une température voisine de 20 °C en présence de potasse en pastilles. On obtient ainsi 2,4 g de produit qui sont réunis à 0,9 g de produit préparé de la même

façon dans une opération antérieure et chromatographiés sur une colonne de 2,8 cm de diamètre contenant 35 g de silice (0,063–0,2 mm) en recueillant des fractions de 500 $cm^3$. Les 2 premières fractions provenant de l'élution par du chlorure de méthylène pur, ainsi que les 2 suivantes provenant de l'élution par un mélange de chlorure de méthylène et de méthanol (97,5–2,5 en volumes) sont éliminées. La cinquième et la sixième fraction provenant de l'élution par un mélange de chlorure de méthylène et de méthanol (95–5 en volumes) ainsi que la septième fraction provenant de l'élution par un mélange de chlorure de méthylène et de méthanol (90–10 en volumes) sont réunies et concentrées à sec sous pression réduite (20 mm de mercure; 2,7 kPa) à une température voisine de 40 °C. On obtient ainsi 3,1 g de produit qui sont dissous dans 900 $cm^3$ d'acétonitrile bouillant. La solution est additionnée de 0,3 g de noir décolorant et filtrée à chaud. Après refroidissement à une température voisine de 4 °C pendant 2 heures, les cristaux apparus sont séparés par filtration, lavés deux fois par 60 $cm^3$ au total d'acétonitrile et séchés sous pression réduite (20 mm de mercure; 2,7 kPa) à une température voisine de 20 °C en présence de potasse en pastilles. On obtient ainsi 2,1 g de [(pyridyl-3)-2 vinyl]-5 1H,3H-pyrrolo[1,2-c] thiazolecarboxamide-7 fondant à 242 °C.

Le mélange (69–31) de cyano-6 et cyano-7 [(pyridyl-3)-2 vinyl]-5 1H,3H-pyrrolo[1,2-c] thiazole peut être préparé comme décrit à l'exemple 5.

Exemple d'application 15

Une suspension de 3,6 g de (pyridyl-3)-5 1H,3H-pyrrolo [1,2-c] thiazolecarbonitrile-7, de 3,8 g d'éthanolamine et de 0,1 g de chlorure de lithium est chauffée à une température voisine de 122 °C pendant 22 heures. On ajoute alors au mélange réactionnel 1,9 g d'éthanolamine et 0,1 g de chlorure de lithium et on poursuit le chauffage pendant encore 26 heures. Après refroidissement du mélange réactionnel à une température voisine de 20 °C, on observe une prise en masse. On ajoute alors 25 $cm^3$ d'éthanol pour déliter les cristaux. Après 30 minutes d'agitation à une température voisine de 20 °C, les cristaux sont séparés par filtration, lavés 2 fois par 10 $cm^3$ au total d'éthanol et 3 fois par 75 $cm^3$ au total d'oxyde d'isopropyle puis séchés sous pression réduite (20 mm de mercure; 2,7 kPa) à une température voisine de 20 °C en présence de potasse en pastilles. On obtient ainsi 3,1 g de produit brut fondant à 150 °C. Ce produit est dissous dans 40 $cm^3$ d'éthanol bouillant. La solution obtenue est additionnée de 0,5 g de noir décolorant et filtrée à chaud. Le filtrat est refroidi à une température voisine de 4 °C pendant 1 heure. Les cristaux apparus sont séparés par filtration, lavés 2 fois par 10 $cm^3$ au total d'éthanol et 3 fois par 45 $cm^3$ au total d'oxyde d'isopropyle puis séchés sous pression réduite (20 mm de mercure; 2,7 kPa) à une température voisine de 20 °C en présence de potasse en pastilles. On obtient ainsi 2 g de N-[(hydroxy-2 éthyl) amino-2 éthyl] (pyridyl-3)-5 1H,3H-pyrrolo[1,2-c] thiazolecarboxamide-7 sous forme de cristaux jaune pâle fondant à 163 °C.

Le (pyridyl-3)-5 1H,3H-pyrrolo[1,2-c] thiazolecarbonitrile-7 est préparé comme à l'exemple 3.

Exemple d'application 16

Une suspension de 12,3 g de potasse en poudre et de 11,3 g d'un mélange (dans la proportion 20–80) de cyano-6 et de cyano-7 phényl-3 (pyridyl-3)-5 1H,3H-pyrrolo[1,2-c] thiazole dans 260 $cm^3$ d'alcool tert-butylique est chauffée au reflux pendant 3 heures. La suspension est agitée pendant 16 heures à une température voisine de 20 °C puis est concentrée à sec sous pression réduite (20 mm de mercure; 2,7 kPa) à une température voisine de 60 °C. Le résidu obtenu est mis en suspension dans 300 $cm^3$ d'eau distillée et est extrait 3 fois par 750 $cm^3$ au total d'acétate d'éthyle. Les extraits organiques sont réunis, lavés 3 fois par 300 $cm^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 60 °C. On obtient ainsi 9,2 g de produit brut. Ce produit est chromatographié sur une colonne de 6 cm de diamètre contenant 480 g de silice (0,04–0,063 mm). On élue par un mélange d'acétate d'éthyle et de méthanol (95–5 en volumes) sous une pression de 0,5 bar (51 kPa) en recueillant des fractions de 200 $cm^3$. Les 10 premières fractions sont éliminées. Les 12 fractions suivantes sont réunies et concentrées à sec sous pression réduite (20 mm de mercure; 2,7 kPa) à une température voisine de 60 °C. On obtient ainsi 7 g de produit. Ce produit est dissous dans 140 $cm^3$ d'éthanol. La solution obtenue est additionnée de 3,1 $cm^3$ d'une solution éthanolique d'acide chlorhydrique 2 N et agitée à une température voisine de 20 °C pendant 15 minutes. Les cristaux apparus sont séparés par filtration, lavés 2 fois par 50 $cm^3$ au total d'éthanol et 3 fois par 75 $cm^3$ au total d'éther diéthylique et séchés sous pression réduite (20 mm de mercure; 2,7 kPa) à une température voisine de 20 °C en présence de potasse en pastilles. On obtient ainsi 6 g de chlorhydrate de phényl-3 (pyridyl-3)-5 1H,3H-pyrrolo[1,2-c] thiazolecarboxamide-7 sous forme de cristaux jaunes fondant à 250 °C.

Le mélange (20–80) de cyano-6 et de cyano-7 phényl-3 (pyridyl-3)-5 1H,3H-pyrrolo[1,2-c] thiazole peut être préparé comme décrit à l'exemple 7.

Exemple d'application 17

Une suspension de 20,4 g de potasse en poudre et de 17,6 g d'un mélange (dans la proportion 50–50) de cyano-6 et de cyano-7 méthyl-3 (pyridyl-3)-5 1H,3H-pyrrolo[1,2-c] thiazole dans 200 $cm^3$ d'alcool tert-butylique est chauffée au reflux pendant 1 heure et 20 minutes. Le solvant est évaporé sous pression réduite (20 mm de mercure; 2,7 kPa) à une température voisine de 50 °C. Le résidu est additionné d'une mélange de 200 $cm^3$ d'eau distillée et de 100 $cm^3$ de chlorure de méthylène. La phase organique est séparée par décantation et la phase aqueuse est extraite 4 fois par 400 $cm^3$ au

total de chlorure de méthylène. Les extraits organiques sont réunis, lavés 5 fois par 750 $cm^3$ au total d'eau distillée, séchés sur du sulfate de magnésium anhydre, filtrés et concentrés à sec sous pression réduite (20 mm de mercure; 2,7 kPa) à une température voisine de 50 °C. On obtient ainsi 19 g de produit. Ce produit est dissous dans 50 $cm^3$ de chlorure de méthylène. Des cristaux apparaissent; ils sont séparés par filtration, lavés 3 fois par 15 $cm^3$ au total de chlorure de méthylène et séchés sous pression réduite (20 mm de mercure; 2,7 kPa) en présence de potasse en pastilles. On obtient ainsi 6,1 g de produit fondant à 170 °C. Le filtrat est chromatographié sur une colonne de 6 cm de diamètre contenant 480 g de silice (0,04–0,063 mm). On élue par des mélanges d'acétate d'éthyle et de méthanol sous une pression de 0,5 bar (51 kPa) en recueillant des fractions de 250 $cm^3$. Les 13 premières fractions provenant de l'élution par un mélange d'acétate d'éthyle et de méthanol (97,5–2,5 en volumes) sont éliminées. Les 3 fractions suivantes provenant de l'élution par un mélange d'acétate d'éthyle et de méthanol (95–5 en volumes) et les trois fractions suivantes provenant de l'élution par un mélange d'acétate d'éthyle et de méthanol (95–5 en volumes) sont réunies et concentrées à sec sous pression réduite (20 mm de mercure; 2,7 kPa) à une température voisine de 50 °C. On obtient ainsi 3,1 g de produit que l'on réunit aux 6,1 g précédemment obtenus et que l'on dissout dans 90 $cm^3$ d'isopropanol bouillant. La solution obtenue est additionnée de 0,5 g de noir décolorant et filtrée à chaud. Le filtrat est refroidi à une température voisine de 4 °C pendant 1 heure. Les cristaux apparus sont séparés par filtration, lavés 3 fois par 6 $cm^3$ au total d'isopropanol refroidi à une température voisine de 4 °C et 3 fois par 30 $cm^3$ au total d'éther diéthylique et séchés sous pression réduite (20 mm de mercure; 2,7 kPa) à une température voisine de 20 °C en présence de potasse en pastilles. On obtient ainsi 5,6 g de méthyle-3 (pyridyl-3)-5 1H,3H-pyrollo [1,2-c] thiazolecarboxamide-7 sous forme de cristaux crème fondant à 170 °C.

Le mélange (50–50) de cyano-6 et de cyano-7 méthyl-3 (pyridyl-3)-5 1H,3H-pyrrolo[1,2-c] thiazole peut être obtenu comme décrit à l'exemple 6.

Exemple d'application 18

A une suspension de 51,5 g de chlorhydrate d'amino-2 éthanethiol dans 250 $cm^3$ d'éthanol on ajoute en 15 minutes à une température voisine de 10 °C 50,1 g de triéthylamine. La suspension obtenue est agitée à une température voisine de 10 °C pendant 15 minutes puis filtrée. Le filtrat est additionné de 20,5 g de (pyridyl-3)-5 1H,3H-pyrrolo[1,2-c] thiazolecarbonitrile-7 et la suspension obtenue est chauffée à l'ébullition pendant 22 heures. La solution obtenue est refroidie à une température voisine de 4 °C et les cristaux apparus sont séparés par filtration, lavés 3 fois par 75 $cm^3$ au total d'éthanol refroidi à une température voisine de 4 °C et 4 fois par 100 $cm^3$ au total d'éther diéthylique puis séchés sous pression réduite (20 mm de mercure; 2,7 kPa) à une température voisine de 20 °C en présence de potasse en pastilles. On obtient ainsi 25 g de produit fondant à 136 °C. Ce produit est réuni avec 1,5 g de produit provenant d'une autre opération antérieure et dissous dans 400 $cm^3$ d'éthanol bouillant. La solution obtenue est additionnée de 0,5 g de noir décolorant et filtrée à chaud. Le filtrat est refroidi à température voisine de 4 °C pendant 1 heure. Les cristaux apparus sont séparés par filtration, lavés 3 fois par 7 $cm^3$ au total d'éthanol et 3 fois par 150 $cm^3$ au total d'éther diéthylique et séchés sous pression réduite (20 mm de mercure; 2,7 kPa) à une température voisine de 20 °C en présence de potasse en pastilles. On obtient ainsi 23 g de produit fondant à 124 °C. Ce produit est dissous dans 300 $cm^3$ d'acétonitrile bouillant. La solution obtenue est additionnée de 0,5 g de noir décolorant et filtrée à chaud. Le filtrat est refroidi à une température voisine de 4 °C pendant 1 heure. Les cristaux apparus sont séparés par filtration, lavés 3 fois par 75 $cm^3$ au total d'acétonitrile refroidi à une température voisine de 4 °C et 3 fois par 150 $cm^3$ au total d'éther diéthylique puis séchés sous pression réduite (20 mm de mercure; 2,7 kPa) à une température voisine de 20 °C en présence de potasse en pastilles. On obtient ainsi 20,1 g de (dihydro-4,5 thiazolyl-2)-7 (pyridyl-3)-5 1H,3H-pyrrolo[1,2-c] thiazole sous forme de cristaux jaune orangé fondant à 124 °C.

Le (pyridyl-3)-5 1H,3H-pyrrolo[1,2-c] thiazolecarbonitrile-7 est préparé comme à l'exemple 3.

Exemple d'application 19

On chauffe pendant 13 heures à une température voisine de 80 °C une suspension de 5,2 g de (pyridyl-3)-3 tétrahydro-5,6,7,8 indolizinecarbonitrile-1 et de 7,7 g de potasse en poudre dans 120 $cm^3$ d'alcool tert-butylique. La suspension est ensuite concentrée à sec sous pression réduite (20 mm de mercure; 2,7 kPa) à une température voisine de 60 °C. Le résidu est repris avec 200 $cm^3$ d'eau distillée et la suspension obtenue est agitée à une température voisine de 20 °C pendant 1 heure. Les cristaux apparus sont séparés par filtration, lavés 5 fois par 250 $cm^3$ au total d'eau distillée et séchés sous pression réduite (20 mm de mercure; 2,7 kPa) à une température voisine de 20 °C en présence de potasse en pastilles. On obtient ainsi 4,1 g de produit brut fondant à 180 °C. Ce produit, réuni à 1,1 g de produit préparé de la même façon dans une autre opération, est dissous dans 160 $cm^3$ d'acétonitrile bouillant. La solution trouble obtenue est additionnée de 0,5 g de noir décolorant et filtrée à chaud. Le filtrat est refroidi à une température voisine de 4 °C pendant 2 heures. Les cristaux apparus sont séparés par filtration, lavés 3 fois par 75 $cm^3$ au total d'acétonitrile et 3 fois par 75 $cm^3$ au total d'oxyde d'isopropyle puis séchés sous pression réduite (20 mm de mercure; 2,7 kPa) à une température voisine de 20 °C en présence de potasse en pastilles. On obtient ainsi 4 g de (pyridyl-3)-3 tétrahydro-5,6,7,8 indolizinecarboxamide-1 sous forme de cristaux beige clair fondant à 184 °C.

Le (pyridyl-3)-3 tétrahydro-5,6,7,8 indolizinecar-

bonitrile-1 peut être préparé comme décrit à l'exemple 8.

En thérapeutique humaine, les produits de formule générale (XIII) définis comme précédemment en A sont particulièrement utiles dans le traitement des affections allergiques et inflammatoires et, d'une façon générale, dans toutes les affections dans lesquelles le rôle physiopathologique du PAF-Acéther peut être incriminé. Les doses dépendent de l'effet recherché et de la durée du traitement; elles sont généralement comprises entre 25 et 100 mg par jour par voie orale, intraveineuse ou par inhalation pour un adulte en une ou plusieurs prises.

En thérapeutique humaine, les produits de formule générale (XIII) définis comme précédemment en B sont particulièrement utiles dans le traitement prophylactique et thérapeutique des affections thrombotiques. Les doses dépendent de l'effet recherché et de la durée du traitement; elles sont généralement comprises entre 100 et 1000 mg par jour par voie orale pour un adulte en une ou plusieurs prises et entre 10 et 100 mg par voie parentérale pour un adulte en une ou plusieurs injections.

D'une façon générale, le médecin déterminera la posologie qu'il estime la plus appropriée en fonction de l'âge, du poids et de tous les autres facteurs propres au sujet à traiter.

**Revendications pour les Etats contractants BE, CH, DE, FR, GB, IT, LI, LU, NL, SE**

1. Dérivé du pyrrole orthocondensé caractérisé en ce qu'il répond à la formule générale:

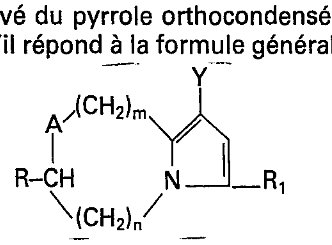

(I)

dans laquelle

a) soit Y représente un radical carboxy ou un radical de formule générale:

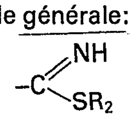

(II)

dans laquelle $R_2$ représente un radical alcoyle ou benzyle, A représente un atome de soufre, m représente le nombre 1, n représente le nombre zéro, R représente un radical pyridyl-3 et $R_1$ représente un atome d'hydrogène,

b) soit Y représente un radical cyano, carboxy ou un radical de formule générale (II) défini comme précédemment, A représente un atome de soufre ou d'oxygène ou un radical méthylène, m représente le nombre 1 ou 2 et n représente le nombre 0, 1 ou 2, étant entendu que la somme m + n est égale à 1, 2 ou 3, R représente un atome d'hydrogène ou un radical alcoyle ou phényle (éventuellement substitué par un atome d'halogène ou un radical alcoyle, alcoyloxy ou trifluorométhyle) et $R_1$ représente un radical de formule générale:

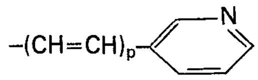

(III)

dans laquelle p représente le nombre zéro ou 1, étant entendu que, dans les définitions qui précèdent et celles qui suivront, les radicaux alcoyle ou portions alcoyle sont en chaîne droite ou ramifiée et contiennent 1 à 4 atomes de carbone, ainsi que ses sels d'additions avec les acides et, le cas échéant, ses sels métalliques et ses sels d'addition avec les bases azotées.

2. Procédé de préparation d'un produit selon la revendication 1 dans la formule duquel Y représente un radical cyano et les autres symboles sont définis comme à la revendication 1 en b), caractérisé en ce que l'on fait agir le chloro-2 acrylonitrile sur un produit de formule générale:

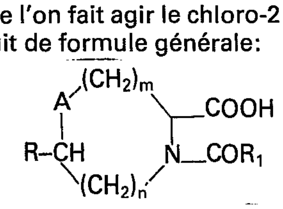

dans laquelle les différents symboles sont définis comme à la revendication 1 en b), puis isole le produit et le transforme éventuellement en un sel d'addition avec un acide.

3. Procédé de préparation d'un produit selon la revendication 1 dans la formule duquel Y représente un radical carboxy et les autres symboles sont définis comme à la revendication 1, caractérisé en ce que l'on hydrolyse un nitrile de formule générale:

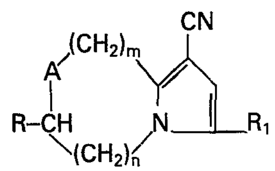

dans laquelle les symboles sont définis comme à la revendication 1, par toute méthode connue de l'homme du métier pour transformer un nitrile en acide sans toucher au reste de la molécule, puis isole le produit obtenu et le transforme éventuellement en un sel d'addition avec un acide, en un sel métallique ou en un sel d'addition avec une base azotée.

4. Procédé de préparation d'un produit selon la revendication 1 dans la formule duquel Y représente un radical de formule générale (II) défini comme à la revendication 1 et les autres symboles sont définis comme la revendication 1, caractérisé en ce que l'on fait agir un halogénure de formule générale:

$R_2Z$

dans laquelle $R_2$ est défini comme à la revendication 1 et Z représente un atome d'halogène, sur un produit de formule générale:

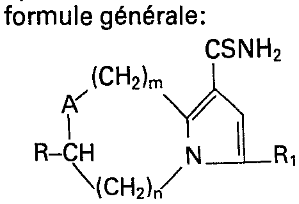

dans laquelle les symboles sont définis comme à la revendication 1, puis isole le produit obtenu et le transforme éventuellement en un sel d'addition avec un acide.

**Revendication pour l'Etat contractant AT**

Procédé de préparation d'un nouveau dérivé du pyrrole orthocondensé de formule générale:

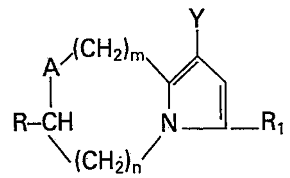

(I)

dans laquelle

a) soit Y représente un radical carboxy ou un radical de formule générale:

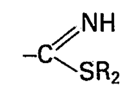

(II)

dans laquelle $R_2$ représente un radical alcoyle ou benzyle, A représente un atome de soufre, m représente le nombre 1, n représente le nombre zéro, R représente un radical pyridyl-3 et $R_1$ représente un atome d'hydrogène,

b) soit Y représente un radical cyano, carboxy ou un radical de formule générale (II) défini comme précédemment, A représente un atome de soufre ou d'oxygène ou un radical méthylène, m représente le nombre 1 ou 2 et n représente le nombre 0, 1 ou 2, étant entendu que la somme m + n est égale à 1, 2 ou 3, R représente un atome d'hydrogène ou un radical alcoyle ou phényle (éventuellement substitué par un atome d'halogène ou un radical alcoyle, alcoyloxy ou trifluorométhyle) et $R_1$ représente un radical de formule générale:

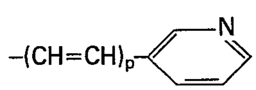

(III)

dans laquelle p représente le nombre zéro ou 1, étant entendu que, dans les définitions qui précèdent et celles qui suivront, les radicaux alcoyle ou portions alcoyle sont en chaîne droite ou ramifiée et contiennent 1 à 4 atomes de carbone, ainsi que ses sels d'additions avec les acides et, le cas échéant, ses sels métalliques et ses sels d'addition avec les bases azotées, caractérisé en ce que

A – Pour la préparation d'un produit de formule générale (I) dans laquelle Y représente un radical cyano et les autres symboles sont définis comme précédemment en b), l'on fait agir le chloro-2 acrylonitrile sur un produit de formule générale:

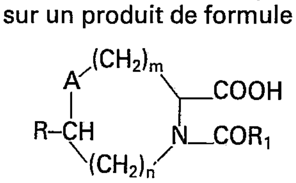

dans laquelle les différents symboles sont définis comme précédemment en b), puis isole le produit et le transforme éventuellement en un sel d'addition avec un acide, ou en ce que

B – Pour la préparation d'un produit de formule générale (I) dans laquelle Y représente un radical carboxy et les autres symboles sont définis comme précédemment l'on hydrolyse un nitrile de formule générale:

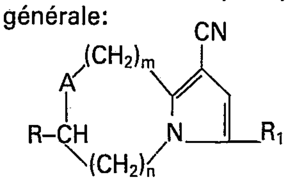

dans laquelle les symboles sont définis comme à précédemment, par toute méthode connue de l'homme du métier pour transformer un nitrile en acide sans toucher au reste de la molécule, puis isole le produit obtenu et le transforme éventuellement en un sel d'addition avec un acide, en un sel métallique ou en un sel d'addition avec une base azotée, ou en ce que

C – Pour la préparation d'un produit de formule générale (I) dans laquelle Y représente un radical de formule générale (II) défini comme précédemment et les autres symboles sont définis comme précédemment l'on fait agir un halogénure de formule générale:

$R_2Z$

dans laquelle $R_2$ est défini comme précédemment et Z représente un atome d'halogène, sur un produit de formule générale:

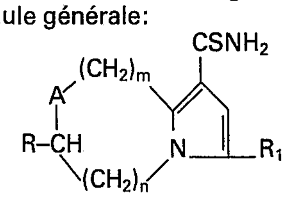

dans laquelle les symboles sont définis comme précédemment, puis isole le produit obtenu et le transforme éventuellement en un sel d'addition avec un acide.

**Patentansprüche für die Vertragsstaaten BE, CH, DE, FR, GB, IT, LI, LU, NL, SE**

1. Derivat des orthokondensierten Pyrrols, dadurch gekennzeichnet, dass es der allgemeinen Formel:

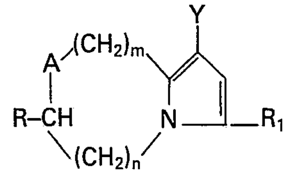

(I)

entspricht, worin

a) entweder Y einen Carboxyrest oder einen Rest der allgemeinen Formel:

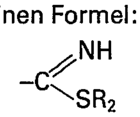

(II)

bedeutet, worin $R_2$ einen Alkyl- oder Benzylrest darstellt, A ein Schwefelatom bedeutet, m die Zahl 1 bedeutet, n die Zahl 0 ist, R einen Pyridyl-3-Rest darstellt und $R_1$ ein Wasserstoffatom ist,

b) oder Y einen Cyano- oder Carboxyrest oder einen Rest der allgemeinen Formel (II) wie vorste-

hend definiert bedeutet, A ein Schwefel- oder Sauerstoffatom oder einen Methylenrest darstellt, m die Zahl 1 oder 2 ist und n die Zahl 0, 1 oder 2 ist, wobei jedoch die Summe von m + n gleich 1, 2 oder 3 ist, R ein Wasserstoffatom oder einen Alkyl- oder Phenylrest bedeutet (gegebenenfalls substituiert durch ein Halogenatom oder einen Alkyl-, Alkyloxy- oder Trifluormethylrest) und $R_1$ einen Rest der allgemeinen Formel:

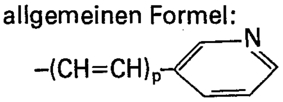

(III)

darstellt, worin p die Zahl 0 oder 1 bedeutet, wobei jedoch in den vorstehenden und den folgenden Definitionen die Alkylreste oder Alkylteile in gerader oder verzweigter Kette sind und 1 bis 4 Kohlenstoffatome enthalten, sowie seine Additionssalze mit Säuren und gegebenenfalls seine Metallsalze und seine Additionssalze mit Stickstoffbasen.

2. Verfahren zur Herstellung eines Produkts gemäss Anspruch 1, in dessen Formel Y einen Cyanorest darstellt und die übrigen Symbole wie in Anspruch 1 unter b) definiert sind, dadurch gekennzeichnet, dass man 2-Chloracrylnitril auf ein Produkt der allgemeinen Formel:

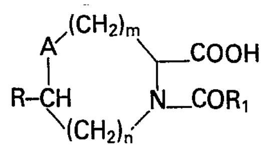

worin die verschiedenen Symbole wie in Anspruch 1 unter b) definiert sind, einwirken lässt, das Produkt dann isoliert und es gegebenenfalls in ein Additionssalz mit einer Säure überführt.

3. Verfahren zur Herstellung eines Produkts gemäss Anspruch 1, in dessen Formel Y einen Carboxyrest bedeutet und die übrigen Symbole wie in Anspruch 1 definiert sind, dadurch gekennzeichnet, dass man ein Nitril der allgemeinen Formel:

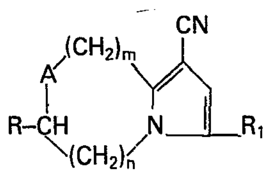

worin die Symbole wie in Anspruch 1 definiert sind, durch jede Methode, die dem Fachmann zur Umwandlung eines Nitrils in eine Säure bekannt ist, ohne dass der Rest des Moleküls berührt wird, hydrolysiert, das erhaltene Produkt dann isoliert und es gegebenenfalls in ein Additionssalz mit einer Säure in ein Metallsalz oder ein Additionssalz mit einer Stickstoffbase überführt.

4. Verfahren zur Herstellung eines Produkts gemäss Anspruch 1, in dessen Formel Y einen Rest der allgemeinen Formel (II), definiert wie in Anspruch 1, bedeutet, und die übrigen Symbole wie in Anspruch 1 definiert sind, dadurch gekennzeichnet, dass man ein Halogenid der allgemeinen Formel:

$R_2Z$

worin $R_2$ wie in Anspruch 1 definiert ist und Z ein Halogenatom bedeutet, auf ein Produkt der allgemeinen Formel:

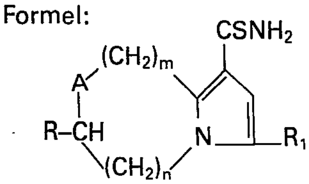

worin die Symbole wie in Anspruch 1 definiert sind, einwirken lässt, das erhaltene Produkt dann isoliert und es gegebenenfalls in ein Additionssalz mit einer Säure überführt.

**Patentanspruch für den Vertragsstaat AT**

Verfahren zur Herstellung eines neuen orthokondensierten Pyrrolderivats der allgemeinen Formel:

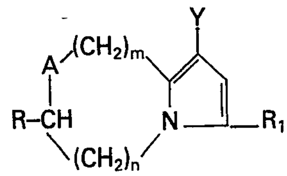

(I)

in welcher

a) entweder Y einen Carboxyrest oder einen Rest der allgemeinen Formel:

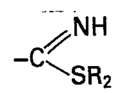

(II)

darstellt, in welcher $R_2$ einen Alkyl- oder Benzylrest darstellt, A ein Schwefelatom bedeutet, m für die Zahl 1 steht, n die Zahl Null darstellt, R einen 3-Pyridylrest darstellt und $R_1$ ein Wasserstoffatom darstellt,

b) oder Y einen Cyano-, Carboxyrest oder einen Rest der allgemeinen Formel (II) wie oben angegeben darstellt, A ein Schwefel- oder Sauerstoffatom oder einen Methylenrest darstellt, m die Zahl 1 oder 2 darstellt und n für die Zahl 0, 1 oder 2 steht, wobei selbstverständlich die Summe von m + n gleich 1, 2 oder 3 ist, R ein Wasserstoffatom oder einen Alkyl- oder (gegebenenfalls durch ein Halogenatom oder einen Alkyl-, Alkyloxy- oder Trifluormethylrest substituierten) Phenylrest darstellt und $R_1$ einen Rest der allgemeinen Formel

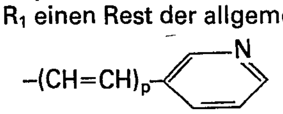

(III)

darstellt, in welcher p die Zahl Null oder 1 bedeutet, wobei selbstverständlich in den obigen und den folgenden Definitionen die Alkylreste oder Alkylteile geradkettig oder verzweigt sind und 1 bis 4 Kohlenstoffatome enthalten, sowie von seinen Säureadditionssalzen und, zutreffendenfalls, von seinen Metallsalzen und seinen Additionssalzen mit Stickstoffbasen, dadurch gekennzeichnet, dass man

A – zur Herstellung einer Verbindung der allgemeinen Formel (I), in welcher Y einen Cyanorest darstellt und die übrigen Symbole die oben unter b) angegebene Bedeutung haben, den 2-Chlor-Acrylnitrilrest auf eine Verbindung der allgemeinen Formel:

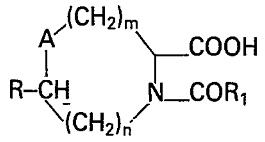

in welcher die verschiedenen Symbole die oben unter b) angegebene Bedeutung haben, einwirken lässt, dann die Verbindung isoliert und sie gegebenenfalls in ein Säureadditionssalz umwandelt, oder dass man

B – zur Herstellung einer Verbindung der allgemeinen Formel (I), in welcher Y einen Carboxyrest darstellt und die übrigen Symbole die obige Bedeutung haben, ein Nitril der allgemeinen Formel:

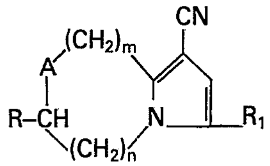

in welcher die Symbole die obige Bedeutung haben, nach irgendeiner dem Fachmann bekannten Methode zur Umwandlung eines Nitrils in eine Säure, wobei der Rest des Moleküls nicht angegriffen wird, hydrolysiert, dann die erhaltene Verbindung isoliert und sie gegebenenfalls in ein Säureadditionssalz, in ein Metallsalz oder in ein Additionssalz mit einer Stickstoffbase umwandelt, oder dass man

C – zur Herstellung einer Verbindung der allgemeinen Formel (I), in welcher Y einen Rest der allgemeinen Formel (II), wie oben angegeben, darstellt und die übrigen Symbole die obige Bedeutung haben, ein Halogenid der allgemeinen Formel:

$R_2Z$

in welcher $R_2$ die obige Bedeutung hat und Z für ein Halogenatom steht, auf eine Verbindung der allgemeinen Formel:

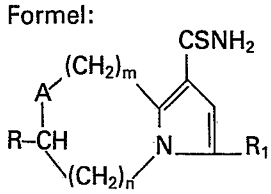

in welcher die Symbole die obige Bedeutung haben, einwirken lässt, dann die erhaltene Verbindung isoliert und sie gegebenenfalls in ein Säureadditionssalz überführt.

**Claims for the Contracting States BE, CH, DE, FR, GB, IT, LI, LU, NL, SE**

1. Ortho-fused pyrrole derivative, characterized in that it corresponds to the general formula:

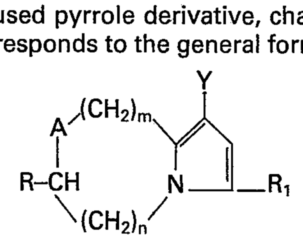

(I)

in which

a) either Y denotes a carboxyl radical or a radical of general formula:

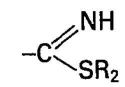

(II)

in which $R_2$ denotes an alkyl or benzyl radical, A denotes a sulphur atom, m denotes the number 1, n denotes the number zero, R denotes a 3-pyridyl radical and $R_1$ denotes a hydrogen atom,

b) or Y denotes a cyano or carboxy radical or a radical of general formula (II) defined as above, A denotes a sulphur or oxygen atom or a methylene radical, m denotes the number 1 or 2 and n denotes the number 0, 1 or 2, on the understanding that the sum (m + n) is equal to 1, 2 or 3, R denotes a hydrogen atom or an alkyl or phenyl radical (optionally substituted with a halogen atom or an alkyl, alkyloxy or trifluoromethyl radical), and $R_1$ denotes a radical of general formula:

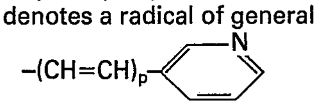

(III)

in which p denotes the number zero or 1, on the understanding that, in the definitions above and those which follow, the alkyl radicals or alkyl portions have linear or branched chains and contain 1 to 4 carbon atoms, as well as its addition salts with acids and, where appropriate, its metal salts and its addition salts with nitrogenous bases.

2. Process for preparing a product according to claim 1, in the formula of which Y denotes a cyano radical and the other symbols are defined as in claim 1 at b), characterized in that 2-chloroacrylonitrile is reacted with a product of general formula:

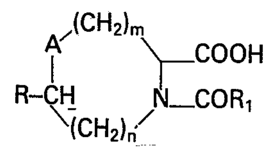

in which the various symbols are defined as in claim 1 at b), and the product is then isolated and optionally converted to an addition salt with an acid.

3. Process for preparing a product according to claim 1, in the formula of which Y denotes a carboxy radical and the other symbols are defined as in claim 1, characterized in that a nitrile of general formula:

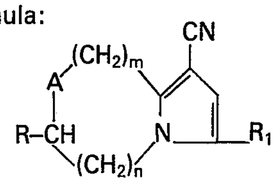

in which the symbols are defined as in claim 1, is hydrolyzed by any method known to those versed in the art for converting a nitrile to an acid without affecting the remainder of the molecule, and the product obtained is then isolated and optionally converted to an addition salt with an acid, to a metal salt or to an addition salt with a nitrogenous base.

4. Process for preparing a product according to claim 1, in the formula of which Y denotes a radical of the general formula (II) defined as in claim 1 and the other symbols are defined as in claim 1, characterized in that a halide of general formula:

$R_2Z$

in which $R_2$ is defined as in claim 1 and Z denotes a halogen atom, is reacted with a product of general formula:

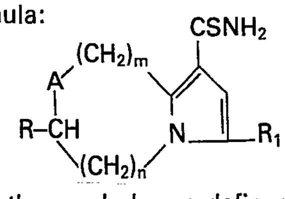

in which the symbols are defined as in claim 1, and the product obtained is then isolated and optionally converted to an addition salt with an acid.

**Claim for the Contracting State AT**

Process for preparing a new ortho-fused pyrrole derivative of general formula:

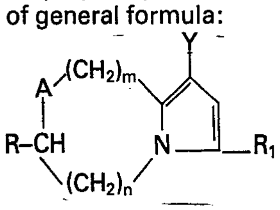

(I)

in which

a) either Y denotes a carboxyl radical or a radical of general formula:

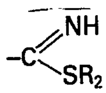

(II)

in which $R_2$ denotes an alkyl or benzyl radical, A denotes a sulphur atom, m denotes the number 1, n denotes the number zero, R denotes a 3-pyridyl radical and $R_1$ denotes a hydrogen atom,

b) or Y denotes a cyano or carboxy radical or a radical of general formula (II) defined as above, A denotes a sulphur or oxygen atom or a methylene radical, m denotes the number 1 or 2 and n denotes the number 0, 1 or 2, on the understanding that the sum (m + n) is equal to 1, 2 or 3, R denotes a hydrogen atom or an alkyl or phenyl radical (optionally substituted with a halogen atom or an alkyl, alkyloxy or trifluoromethyl radical), and $R_1$ denotes a radical of general formula:

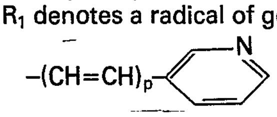

(III)

in which p denotes the number zero or 1, on the understanding that, in the definitions above and those which follow, the alkyl radicals or alkyl portions have linear or branched chains and contain 1 to 4 carbon atoms, as well as its addition salts with acids and, where appropriate, its metal salts and its addition salts with nitrogeneous bases, characterized in that

A – for the preparation of a product of general formula (I) in which Y denotes a cyano radical and the other symbols are defined as above at b), 2-chloroacrylonitrile is reacted with a product of general formula:

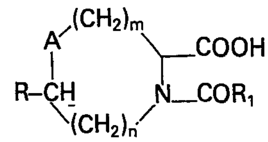

in which the various symbols are defined as above at b), and the product is then isolated and optionally converted to an addition salt with an acid, or in that

B – for the preparation of a product of general formula (I) in which Y denotes a carboxy radical and the other symbols are defined as above, a nitrile of general formula:

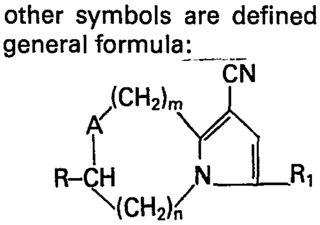

in which the symbols are defined as above, is hydrolyzed by any method known to those versed in the art for converting a nitrile to an acid without affecting the remainder of the molecule, and the product obtained is than isolated and optionally converted to an addition salt with an acid, to a metal salt or to an addition salt with a nitrogenous base, or in that

C – for the preparation of a product of the general formula (I) in which Y denotes a radical of general formula (II) defined as above and the other symbols are defined as above, a halide of general formula:

$R_2Z$

in which $R_2$ is defined as above and Z denotes a halogen atom, is reacted with a product of general formula:

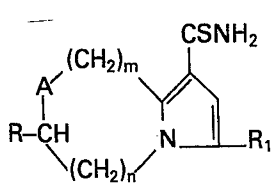

in which the symbols are defined as above, and the product obtained is then isolated and optionally converted to an addition salt with an acid.